# EUROPEAN PATENT APPLICATION

(11) **EP 4 541 896 A1**
(43) Date of publication of application: **23.04.2025**
(21) Application number: 23823787.9
(22) Date of filing: 06.06.2023
(51) Int. Cl.: C12N 15/12, C07K 14/47, C07K 14/705, C12N 1/15, C12N 1/19, C12N 1/21, C12N 5/10, C12N 15/09, C12N 15/63, C12N 15/85, C12P 21/02, C12Q 1/02, C12Q 1/06, C12Q 1/6897, C40B 40/08, G01N 33/53, G01N 33/566

(54) **METHOD FOR ANALYZING G-PROTEIN CONJUGATED RECEPTOR**

(30) Priority: 16.06.2022 JP 2022097443; 16.06.2022 JP 2022097444; 16.06.2022 JP 2022097445; 16.06.2022 JP 2022097446; 16.06.2022 JP 2022097447; 22.12.2022 JP 2022205847
(71) Applicant: Kao Corporation, Tokyo 103-8210 (JP)
(72) Inventor: YOSHIKAWA, Keiichi, Haga-gun, Tochigi 321-3497 (JP)
(74) Representative: Vossius & Partner Patentanwälte Rechtsanwälte mbB
(86) International application number: PCT/JP2023/021052
(87) International publication number: WO 2023/243495

(57) **Abstract**

Provided is an approach which can express and function a G protein-coupled receptor (GPCR). The present invention provides a method for expressing a GPCR polypeptide, comprising expressing in a cell a GPCR polypeptide consisting of an amino acid sequence obtained by, in an amino acid sequence of a GPCR of interest (provided that an olfactory receptor is excluded), altering at least one amino acid residue different from that in a consensus amino acid sequence to an amino acid residue at a position corresponding thereto in the consensus amino acid sequence, wherein the consensus amino acid sequence is an amino acid sequence derived by alignment of the amino acid sequence of the GPCR of interest and amino acid sequences of GPCRs encoded by orthologs of the GPCR of interest in vertebrates.

## Description

### Field of the Invention

The present invention relates to a method for analyzing a G protein-coupled receptor.

### Background of the Invention

As members of the living body, cells recognize changes in the surrounding physiological environment and change their behavior accordingly. Individual organisms utilize their senses to detect changes in the external environment and adopt an adaptive behavior. The basis of biological mechanisms is the appropriate response of individual living units to endogenous semiochemical substances, such as hormones and neurotransmitter substances, or exogenous semiochemical substances, such as taste substances. Receptor proteins present in individual tissue cells are responsible for such chemoreceptions. In recent years, a large number of individual organisms lacking specific receptor genes have been created, demonstrating the necessity of receptors in physiological function. In addition, various drugs which target receptors are being put into practice to regulate physiological function. On the other hand, there are many receptors whose functions are still unknown.

Typical receptors present in living organisms are G protein-coupled receptors (GPCRs). These are characterized by a seven-transmembrane structure, shift to an active structure upon agonist binding, and basically transmit signals through interactions with intracellular G proteins. In humans, there are about 800 types of GPCRs, about half of which are known to be not directly involved in sensory-related physiological functions in the body. Information on human GPCRs, except for olfactory receptors and vomeronasal receptors, can be obtained from the database (G protein-coupled receptors (IUPHAR/BPS Guide to PHARMACOLOGY, http://www.guidetopharmacology.org/GRAC/FamilyDisplayForw ard?familyId=694)). As of November 2017, 134 GPCRs are considered as target molecules for approved drugs in the US (Non Patent Literature 1). In addition, receptors which were once thought to be involved only in sensory functions are actually expressed in tissues other than sensory tissues, and have been shown to be involved in specific physiological functions in the body.

For example, vomeronasal receptors (vomeronasal 1 receptors: VN1Rs) are class A family GPCRs, and there are five types of genes in humans. Based on analysis of homologous genes in mice, they are predicted to be expressed in the nasal cavity and to be responsible for pheromone recognition (Non Patent Literature 2). Only one case of VN1R1 has been reported in humans, in which the function of VN1R1 was analyzed in cultured cells, and it was shown that VN1R1 recognizes low-molecular-weight volatile compounds (Non Patent Literature 2). In addition, in a large-scale survey in Sweden, the genetic polymorphism of VN1R1 was investigated to see what kind of differences in characteristics occur, and, as a result, it was shown that there are differences in female sexual characteristics, suggesting a potential function of VN1R1 (Non Patent Literature 3). As for the other VN1R2 to 5, there are no reports of functional analysis, such as identification of substances to be recognized.

Taste 1 receptors (TAS1Rs), which are taste receptors, are a gene family discovered as GPCRs expressed in the tongue, and the human genome includes three types of genes: TAS1R1, TAS1R2, and TAS1R3, all of which are classified into class C type GPCRs. In the taste buds, TAS1R1 and TAS1R2 are expressed in different taste cells and both co-express TAS1R3. Class C type GPCRs, including metabolic glutamate receptors, are known to function by forming dimers, and TAS1Rs are no exception. When TAS1R3 is co-expressed with either TAS1R1 or TAS1R2, HEK293 cells respond to taste substances. A TAS1R1/TAS1R3 complex and a TAS1R2/TAS1R3 complex receive umami and sweetness substances, respectively. It has been reported that a TAS1R2/TAS1R3 complex are expressed in cultured cells and screening is carried out to thereby identify substances which function as allosteric modulators of the complex and enhance sweetness (Non Patent Literature 4). Similarly, when a TAS1R1/TAS1R3 complex is expressed, it is also possible to obtain evaluation results that inosinic acid, which enhances umami, increases the activity of the complex (Non Patent Literature 5). If these TAS1R proteins can be acquired as more stable proteins or expressed in larger amounts on the membranes of cultured cells, it will be possible to efficiently identify materials which enhance umami and sweetness.

Taste 2 receptors (TAS2Rs), which are also taste receptors, are a gene family discovered as bitterness receptors expressed in the tongue, and the human genome includes 25 types of genes. They are classified into class A type GPCRs and shown to recognize various bitterness substances; however, human TAS2R41, TAS2R42, TAS2R45, TAS2R48, and TAS2R60 have not been successfully analyzed functionally, and it is unclear what substance they recognize. Patent Literature 1 discloses a fusion protein of TAS2R with a G protein as a device for efficient functional analysis of TAS2R to enable bitterness evaluation and identification of bitterness regulatory substances by using TAS2R. The expression of TAS2Rs is not limited to the tongue, but is also found in the respiratory, cardiovascular, and nervous systems, indicating that TAS2Rs play various physiological roles in addition to bitterness reception on the tongue (Non Patent Literature 6). In addition, their expression is also found in cancerous tissue cells, such as ovarian cancer and prostate cancer.

Trace amine-associated receptors (TAARs) are class A family GPCRs, and composed of six types of genes in humans. TAAR1, which was first discovered, has been shown to be responsible for recognition of neurotransmitter substances in the brain. Therefore, TAAR1 has been investigated in relation to schizophrenia, depression, addiction, and Parkinson's disease, and there are efforts to use its agonist for the treatment of neuropathic pain (Patent Literature 2). On the other hand, other TAARs except for TAAR1 have been shown to be highly expressed in the olfactory epithelium, to selectively and sensitively recognize volatile amines, and to generate odor sensation (Non Patent Literature 7).

Mas-related G protein-coupled receptors (Mrgprs) are class A family GPCRs, and there are 10 types of genes in humans. Their expression is found in the sensory nervous system and related tissues, for example, in the peripheral skin, and causes itching and pain sensing by recognizing agonists (Non Patent Literature 8). Further, in mice, based on the fact that MrgprB4-expressing sensory neurons are involved in pleasant tactile sensation, efforts to search for pleasant emotion enhancers targeting MrgprB4 are disclosed (Patent Literature 3). More recently, it has been suggested that MrgprE and MrgprF among Mrgprs are expressed in various tissues other than the sensory nerve, such as the ileum, and play a variety of physiological functions; however, there are few reports of agonists (Non Patent Literature 9).

In general, functional analysis of GPCRs is the most widely used method because it is simple and easy to express GPCRs in cultured cells. Analytical methods have been devised in a wide variety of ways. Nevertheless, many of them have not yet been successfully functionally analyzed. GPCRs which have not been successfully functionally analyzed and for which it is not known what kinds of molecules are recognized as ligands are generally denoted as GPR (G protein-coupled receptor) X (X is an arbitrary number). One of the reasons for the delay in the identification of ligands for these GPRs is that even if GPCRs of interest are tried to be expressed in cultured cells, the cultured cells, which differ from native tissue cells, lack factors to stably express the GPCRs on cell membranes.

(Patent Literature 1) JP-B-6924590
(Patent Literature 2) JP-A-2019-517524
(Patent Literature 3) JP-A-2019-118340

(Non Patent Literature 1) Sriram K, Insel PA. Mol Pharmacol. 93(4):251-258 (2018)
(Non Patent Literature 2) Shirokova E, et al. FASEB J. 22(5):1416-25 (2008)
(Non Patent Literature 3) Henningsson, S., Hovey, D., Vass, K. et al. Transl Psychiatry 7, e1102 (2017)
(Non Patent Literature 4) Servant G, Tachdjian C, Tang XQ, Werner S, Zhang F, Li X, Kamdar P, Petrovic G, Ditschun T, Java A, Brust P, Brune N, DuBois GE, Zoller M, Karanewsky DS. Proc Natl Acad Sci U S A. 107(10):4746-51 (2010)
(Non Patent Literature 5) Nelson, G., Chandrashekar, J., Hoon, M. et al. Nature 416, 199-202 (2002)
(Non Patent Literature 6) Tuzim K., Korolczuk A. J Transl Med 19, 440 (2021)
(Non Patent Literature 7) Liberles S.D. Curr Opin Neurobiol. 34:1-7 (2015)
(Non Patent Literature 8) Liu Q, et al. Cell 139(7):1353-1365 (2009)
(Non Patent Literature 9) Juan M. et al. Neuroscience Letters, Volume 748, 2021

### Summary of the Invention

The present invention provides the following 1) to 13).
1) A method for expressing a GPCR polypeptide, comprising:
   expressing, in a cell, a GPCR polypeptide consisting of an amino acid sequence obtained by, in an amino acid sequence of a GPCR of interest (provided that an olfactory receptor is excluded), altering at least one amino acid residue different from that in a consensus amino acid sequence to an amino acid residue at a position corresponding thereto in the consensus amino acid sequence, wherein
   the consensus amino acid sequence is an amino acid sequence derived by alignment of the amino acid sequence of the GPCR of interest and amino acid sequences of GPCRs encoded by orthologs of the GPCR of interest in vertebrates.
2) A method for expressing a GPCR polypeptide, comprising
   expressing, a cell, a GPCR polypeptide consisting of an amino acid sequence obtained by, in an amino acid sequence of a GPCR of interest, altering at least one amino acid residue different from that in a consensus amino acid sequence to an amino acid residue at a position corresponding thereto in the consensus amino acid sequence, wherein
   the GPCR polypeptide consists of an amino acid sequence obtained by, in the amino acid sequence of SEQ ID NO: 36 of a human TAAR6, altering at least one amino acid residue different from that in the consensus amino acid sequence of SEQ ID NO: 143 to an amino acid residue at a position corresponding thereto in the consensus amino acid sequence.
3) A method for measuring a response of a GPCR of interest, comprising
   measuring a response of the GPCR polypeptide expressed by the method according to 1) or 2).
4) A method for searching for a ligand for a GPCR of interest, comprising:
   measuring a response of the GPCR polypeptide expressed by the method according to 1) or 2) in the presence of a test substance; and
   selecting a test substance to which the GPCR polypeptide has responded.
5) A method for evaluating and/or selecting a control agent for recognition of a ligand for a GPCR of interest, comprising:
   adding a test substance and the ligand for a GPCR of interest to the GPCR polypeptide expressed by the method according to 1) or 2); and
   measuring a response of the GPCR polypeptide to the ligand.
6) A method for evaluating taste, comprising:
   adding a test substance to the GPCR polypeptide expressed by the method according to 1) or 2); and
   measuring a response of the GPCR polypeptide to the test substance, wherein
   the GPCR polypeptide is a taste receptor polypeptide.
7) A method for evaluating and/or selecting a suppressor of odor of a ligand for a GPCR of interest, comprising:
   adding a test substance and the ligand for a GPCR of interest to the GPCR polypeptide expressed by the method according to 1) or 2); and
   measuring a response of the GPCR polypeptide to the ligand, wherein
   the GPCR polypeptide is a trace amine-associated receptor polypeptide.
8) A method for evaluating and/or selecting a suppressor of odor of a ligand for a GPCR of interest, comprising:
   adding a test substance to the GPCR polypeptide expressed by the method according to 1) or 2); and
   measuring a response of the GPCR polypeptide to the test substance, wherein
   the GPCR polypeptide is a trace amine-associated receptor polypeptide.
9) An altered GPCR polypeptide consisting of an amino acid sequence obtained by, in an amino acid sequence of a GPCR of interest (provided that an olfactory receptor is excluded), altering at least one amino acid residue different from that in a consensus amino acid sequence to an amino acid residue at a position corresponding thereto in the consensus amino acid sequence, wherein
   the consensus amino acid sequence is an amino acid sequence derived by alignment of the amino acid sequence of the GPCR of interest and amino acid sequences of GPCRs encoded by orthologs of the GPCR of interest in a vertebrate.
10) An altered GPCR polypeptide consisting of an amino acid sequence obtained by, in an amino acid sequence of SEQ ID NO: 36 of human TAAR6, altering at least one amino acid residue different from that in a consensus amino acid sequence of SEQ ID NO: 143 to an amino acid residue at a position corresponding thereto in the consensus amino acid sequence.
11) A polynucleotide encoding the altered GPCR polypeptide according to 9) or 10).
12) A vector or a DNA fragment comprising the polynucleotide according to 11).
13) A transformed cell comprising the vector or the DNA fragment according to 12).

### Brief Description of Drawing

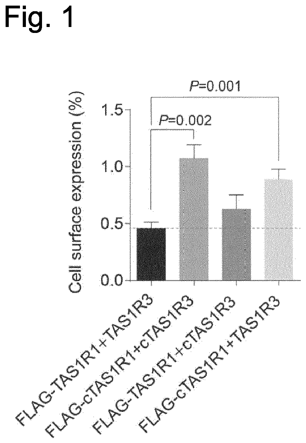

[FIG. 1] FIG. 1 shows membrane expression levels of GPCRs (taste receptors).

### Detailed Description of the Invention

All the Patent Literatures, Non Patent Literatures, and other publications cited in the present specification are incorporated herein by reference in their entirety.

In the present specification, the "G protein-coupled receptor (GPCR)" is a general name for receptors which have a seven-transmembrane structure, bind to ligands to shift to an active structure, and basically transmit signals through interactions with G proteins in cells. Examples of GPCRs include, but are not limited to, vomeronasal receptors, taste receptors, trace amine-associated receptors, and Mas-related G protein-coupled receptors, as well as GPRX (X is any number) with unknown ligands.

In the present specification, the "vomeronasal receptor" refers to VN1R (vomeronasal 1 receptor). For example, the human genome includes 5 types of VN1R genes.

In the present specification, the "taste receptor" refers to a receptor which receives taste molecules in the living body, and includes umami or sweetness receptors belonging to the TAS1R (taste 1 receptor) family which receives umami molecules or sweetness molecules, and bitterness receptors belonging to the TAS2R (taste 2 receptor) family which receives bitterness molecules. For example, the human genome includes 3 types of TAS1R genes. Among these, TASR1 and TAS1R3 form a complex to function as an umami substance receptor, and TAS1R2 and TAS1R3 form a complex to function as a sweetness substance receptor. In addition, the human genome includes 25 types of TAS2R genes.

In the present invention, the "trace amine-associated receptor" refers to TAAR (trace amine-associated receptor). For example, the human genome includes 6 types of TAAR genes.

In the present specification, the "Mas-related G protein-coupled receptor" refers to Mrpgr (Mas-related G protein-coupled receptor). MAS is a G protein-coupled receptor that binds to angiotensin. For example, the human genome includes 10 types of Mrpgr genes (MAS1, MASL1, MrgprD, MrgprE, MrgprF, MrgprG, and MrgprX1-4).

In the present specification, the "olfactory receptor" refers to an olfactory receptor or an odorant receptor. Based on criteria such as overall sequence homology, prediction of a seven-transmembrane region, and whether or not having conserved partial amino acid sequences, the human genome is expected to include about 400 olfactory receptor genes.

In the present specification, the "GPCR polypeptide" refers to a GPCR or a polypeptide functionally equivalent thereto. The polypeptide functionally equivalent to the GPCR refers to a polypeptide which can be expressed on cell membranes, as in the GPCR, is activated through the binding of a ligand, and when activated, has function to transmit signals into cells, such as function to promote GDP/GTP exchange of the coupled G protein α subunit.

In the present specification, the term "functionally expressing" the GPCR polypeptide in cells means that the expressed GPCR polypeptide functions as a corresponding ligand receptor in the cells.

In the present specification, the "agonist" refers to a substance which binds to and activates a receptor. In the present specification, the "antagonist" refers to a substance which binds to a receptor but does not activate the receptor, or suppresses a response of the receptor to an agonist.

In the present specification, the "receptor agonism" refers to binding to a receptor to activate the receptor.

In the present specification, the "odor cross-adaptation (or olfactory cross-adaptation)" regarding target odor refers to a phenomenon in which olfactory sensitivity to a causative substance of the target odor is reduced or changed by receiving in advance odor of a substance different from the causative substance of the target odor and acclimatizing to the odor. The present inventor and so on previously revealed that the "odor cross-adaptation" is a phenomenon based on receptor agonism (WO 2016/194788). Specifically, in the "odor cross-adaptation", a receptor for the causative substance of the target odor responds to a causative substance of different odor prior to responding to the causative substance of the target odor, and is subsequently desensitized so that the olfactory receptor merely low responds to the causative substance of the target odor even when later exposed thereto, resulting in reduction in the intensity or degeneration of the target odor to be recognized by individuals. In the present specification, the mechanism of the odor cross-adaptation caused by such a behavior of a receptor is also referred to as the "odor cross-adaptation ascribable to receptor agonism".

In the present specification, the "suppression ascribable to receptor antagonism" regarding target odor means that an antagonist suppresses a response of a receptor to a substance having the target odor, resulting in the suppression of the target odor to be recognized by individuals.

In the present specification, the identity between nucleotide sequences or amino acid sequences is calculated by the Lipman-Pearson method (Science, 1985, 227: 1435-41). Specifically, the identity is calculated by conducting analysis using homology analysis (Search Homology) program of genetic information processing software Genetyx-Win (Ver. 5.1.1; Software Development K.K.) with Unit size to compare (ktup) set to 2.

In the present specification, the "amino acid residue" means any of 20 amino acid residues constituting a protein: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

In the present specification, the alteration of an amino acid is also represented by [original amino acid, position, altered amino acid] in accordance with the authorized single-letter amino acid abbreviation of IUPAC. For example, the alteration of histidine at position 43 to arginine is represented by "H43R".

In the present specification, the "position corresponding" on an amino acid sequence can be determined by arranging (aligning) a sequence of interest and a reference sequence (in the present invention, an amino acid sequence of the original GPCR) so as to give the largest homology. The alignment of amino acid sequences can be carried out using an algorithm known in the art, and procedures thereof are known to those skilled in the art. The alignment can be performed, for example, by using Clustal W multiple alignment program (Thompson, J.D. et al., 1994, Nucleic Acids Res. 22: 4673-4680) at default setting. Alternatively, Clustal W2 or Clustal omega, a revised version of Clustal W, may be used. Clustal W, Clustal W2, and Clustal omega are available, for example, on the website of Clustal run by University College Dublin [www.clustal.org] or European Bioinformatics Institute (EBI) [www.ebi.ac.uk/index.html] or the website of DNA Data Bank of Japan (DDBJ) run by National Institute of Genetics [www.ddbj.nig.ac.jp/searches-j.html]. A position in the sequence of interest aligned with an arbitrary position in the reference sequence by the alignment mentioned above is regarded as the "position corresponding" to the arbitrary position.

Those skilled in the art can finely adjust and optimize the alignment of the amino acid sequences thus obtained. Such optimized alignment is preferably determined in consideration of similarity between the amino acid sequences, the frequency of a gap to be inserted, and the like. In this context, the similarity between the amino acid sequences refers to, when the two amino acid sequences are aligned, the ratio (%) of the number of positions where the same or similar amino acid residues exist in both the sequences to the number of full length amino acid residues. The similar amino acid residues mean amino acid residues having properties similar to each other in terms of polarity or charge so as to cause so-called conservative substitution, among 20 amino acids constituting a protein. Groups of such similar amino acid residues are well known to those skilled in the art. Examples thereof include, but are not limited to, arginine and lysine or glutamine; glutamic acid and aspartic acid or glutamine; serine and threonine or alanine; glutamine and asparagine or arginine; and leucine and isoleucine.

In addition, the alignment of the amino acid sequences thus obtained can be finely adjusted and optimized, for example, with reference to highly conserved amino acids or amino acid motifs among GPCRs.

In the present specification, the "operable linkage" of a control region such as a promoter to a gene refers to the linkage of the gene and the control region such that the gene can be expressed under the control of the control region. Procedures of the "operable linkage" of the gene and the control region are well known to those skilled in the art.

In the present specification, the terms "upstream" and "downstream" regarding a gene refer to upstream and downstream in the transcriptional directions of the gene. For example, the term "gene located downstream of a promoter" means that the gene resides on a 3' side of the promoter in a DNA sense strand, and the term "upstream of a gene" means a region on a 5' side of the gene in a DNA sense strand.

In the present specification, the "homolog" refers to a homologous gene derived from a common ancestor. The "ortholog", also called "orthologue", refers to a homolog which has diverged after a specification event. The orthologs reside in different organism species and have the same or similar functions. As one example, the ortholog used in the present invention can be a GPCR gene which involves the same name as that a gene of a GPCR of interest and which is of an organism species different from the organism species from which the GPCR of interest is derived, among homologous genes of the gene of the GPCR of interest. When a nomenclature of a GPCR of an organism species is different from a nomenclature in the organism species from which the GPCR of interest is derived, the ortholog may be a GPCR gene having high homology, preferably a GPCR gene having the highest homology, to the gene of the GPCR of interest in the organism species. Alternatively, the ortholog may be a GPCR gene known to be an ortholog of the gene of the GPCR of interest in the organism species. As another example, the ortholog used in the present invention can be a GPCR gene suggested to have the possibility that the gene has diverged after a specification event by phylogenetic tree analysis among the orthologs.

For analysis of G protein-coupled receptors (GPCRs), a method which can express GPCRs efficiently is required.

The present inventor conducted diligent studies on an approach which can express GPCRs efficiently. As a result, the present inventor found that consensus design of a GPCR can improve the membrane expression of the GPCR in cultured cells in comparison with the original GPCR. There have been so far no examples of consensus design of GPCRs, except for olfactory receptors.

The present invention provides an approach which can express GPCRs efficiently. Use of such GPCRs can contribute to the clarification of the function of GPCRs and the identification of control agents for the function.

As shown in Examples mentioned later, the present inventor altered an amino acid sequence of a human GPCR on the basis of a consensus amino acid sequence derived from the amino acid sequence of the human GPCR and amino acid sequences of GPCRs encoded by particular orthologs of the human GPCR, and expressed the obtained GPCR polypeptide in cells. As a result, the present inventor found that the membrane expression of the GPCR polypeptide in the cells can be increased in comparison with the human GPCR before the alteration (Table 7 and Figure 1). Specifically, the GPCR polypeptide is improved in expression stability in comparison with the human GPCR before the alteration. In the present specification, the GPCR before the alteration is also referred to as the "original GPCR"; the alteration of an amino acid sequence of a GPCR on the basis of a consensus amino acid sequence is also referred to as the "consensus design"; and the GPCR obtained by the consensus design is also referred to as the "consensus GPCR" or the "altered GPCR polypeptide".

Accordingly, the consensus design of a GPCR is useful for expressing a GPCR on the cell membranes of cells, particularly, a GPCR which has been inefficient to functionally analyze due to insufficient membrane expression in cultured cells. Thus, in one aspect, the present invention provides a method for expressing a GPCR polypeptide. The expression method of the present invention enables improved expression (e.g., increased expression and stabilized expression) of a GPCR. Therefore, the method is preferably a method for improving expression of a GPCR polypeptide. The method includes expressing, in a cell, a GPCR polypeptide consisting of an amino acid sequence obtained by, in an amino acid sequence of a GPCR of interest, altering at least one amino acid residue different from that in a consensus amino acid sequence to an amino acid residue at a position corresponding thereto in the consensus amino acid sequence, wherein the consensus amino acid sequence is an amino acid sequence derived by alignment of the amino acid sequence of the GPCR of interest and amino acid sequences of GPCRs encoded by orthologs of the GPCR of interest in vertebrates.

In the expression method of the present invention, the GPCR of interest is not particularly limited, may be a GPCR of any organism species, and is preferably a mammalian GPCR, more preferably a human GPCR. The GPCR of interest may be a GPCR possible or inefficient to functionally analyze using cultured cells by a conventional technique. The method of the present invention is more suitably applied to a GPCR inefficient to functionally analyze using cultured cells by a conventional technique.

In the expression method of the present invention, the GPCRs encoded by orthologs of the GPCR of interest in vertebrates are preferably GPCRs selected from the group consisting of GPCRs encoded by orthologs of the GPCR of interest in mammals, Aves, Reptilia, Amphibia, and fish, more preferably GPCRs selected from the group consisting of GPCRs encoded by orthologs of the GPCR of interest in mammals, Aves, Reptilia, and Amphibia, further more preferably GPCRs encoded by orthologs of the GPCR of interest in mammals. The orthologs are not particularly limited and are preferably orthologs having higher homology to the gene of the GPCR of interest. In this context, the mammals refer to organism species belonging to the class *Mammalia* of the phylum *Vertebrata* and are known as approximately 5 500 now-existing species. Examples of the mammals include, but are not limited to, humans, chimpanzees, bonobos, gorillas, Sumatran orangutans, northern white-cheeked gibbons, drills, gelada baboons, rhesus macaques, olive baboons, sooty mangabeys, green monkeys, ashy red colobuses, Angola colobuses, Garnett's greater galagoes, gray mouse lemurs, Coquerel's sifakas, Philippine tarsiers, mice, rats, rabbits, cats, dogs, foxes, raccoon dogs, weasels, tigers, cheetahs, bears, sea lions, earless seals, sea bears, horses, rhinos, camels, pigs, hogs, bovines, goats, sheep, deer, giraffes, hippopotamuses, elephants, pangolins, moles, and bats. The Aves refers to organism species belonging to the class *Aves* of the phylum *Vertebrata*. Examples of the Aves include, but are not limited to, chickens, dabblers, ducks, gooses, turkeys, ostriches, pheasants, doves, parrots, canary-birds, society finches, hummingbirds, manakins, quails, and flycatchers. The Reptilia refers to organism species belonging to the class *Reptilia* of the phylum *Vertebrata.* Examples of the Reptilia include, but are not limited to, tortoises, lizards, gators, iguanas, chameleons, geckos, and snakes. The Amphibia refers to organism species belonging to the class *Amphibia* of the phylum *Vertebrata.* Examples of the Amphibia include, but are not limited to, frogs, newts, and salamanders. The fish refers to organism species belonging to the class *Myxini,* the order *Petromyzontiformes,* the class *Chondrichthyes,* and the class *Osteichthyes* of the phylum *Vertebrata.* Examples of the fish include, but are not limited to, hagfishes, lampreys, sharks, rays, tunas, bonitos, salmons, trout, cods, porgies, flounders, amberjacks, horse mackerels, and mackerels.

In a preferred example of the present embodiment, the orthologs of the GPCR of interest in vertebrates are genes involving the same name as that of the gene of the GPCR of interest among GPCR genes of organism species belonging to the vertebrates. The orthologs can be selected by, for example, the following procedures: database search is performed with a known database such as NCBI BLAST by setting the amino acid sequence of the GPCR of interest to a query sequence. The genes involving the same name as that of the gene of the GPCR of interest are selected from the resulting homologous gene group (e.g., top 500 genes, preferably top 250 genes, more preferably top 100 genes, further more preferably top 50 genes). Further more preferably, genes encoding GPCRs having a certain degree of amino acid sequence identity, for example, an amino acid sequence identity of 65% or more, with the GPCR of interest are selected.

When a plurality of genes derived from the same organism species are selected as the orthologs, only one gene having the highest homology to the gene of the GPCR of interest may be selected. For example, when the GPCR of interest is a human GPCR and a plurality of genes of a non-human organism species are selected as the orthologs, a gene of the organism species having the highest homology to the gene of the human GPCR of interest may be selected. When a nomenclature of a GPCR of an organism species is different from a nomenclature in the organism species from which the GPCR of interest is derived, a gene having high homology, preferably a gene having the highest homology, to the gene of the GPCR of interest in the organism species may be selected as the ortholog. Alternatively, a gene known in the organism species to be an ortholog of the gene of the GPCR of interest may be selected.

The number of types of GPCRs encoded by orthologs of the GPCR of interest in vertebrates is at least 2 types, preferably at least 5 types, more preferably at least 11 types, further more preferably at least 15 types, further more preferably at least 30 types, further more preferably 100 types, in terms of the number of receptors. On the other hand, the upper limit of the number of types is the number of all types of orthologs of the GPCR of interest in vertebrates. The number of types is preferably 500 or less types, more preferably 400 or less types, further more preferably 300 or less types, in terms of the number of receptors. The number of types of GPCRs encoded by orthologs of the GPCR of interest in vertebrates can be, for example, from 2 types to all types of orthologs of the GPCR of interest in vertebrates, from 5 types to all types of orthologs of the GPCR of interest in vertebrates, from 11 types to all types of orthologs of the GPCR of interest in vertebrates, from 5 to 500 types, from 5 to 400 types, from 5 to 300 types, from 11 to 500 types, from 11 to 400 types, from 11 to 300 types, from 15 to 300 types, from 30 to 300 types, or from 100 to 300 types, in terms of the number of receptors.

In the expression method of the present invention, the "consensus amino acid sequence" is an amino acid sequence derived by alignment of the amino acid sequence of the GPCR of interest and the amino acid sequences of GPCRs encoded by orthologs of the GPCR of interest in vertebrates. Specifically, the "consensus amino acid sequence" is an amino acid sequence containing consensus residues identified in accordance with the following criteria (i) to (iii) from the alignment of the amino acid sequence of the GPCR of interest and the amino acid sequences of GPCRs encoded by orthologs of the GPCR of interest in vertebrates.
(i) at each amino acid position of the alignment,
   (i-i) when there exists one amino acid residue which is different from the amino acid residue of the GPCR of interest and has a frequency of appearance of 50% or more, the amino acid residue is identified as a consensus residue,
   (i-ii) when there exist two amino acid residues having a frequency of appearance of 50%, the amino acid residue of the GPCR of interest is identified as a consensus residue,
   (i-iii) when there exists an amino acid residue in the GPCR of interest and there exists no amino acid residue having a frequency of appearance of 40% or more, the absence of a consensus residue is identified,
   (i-iv) when there exists no amino acid residue in the GPCR of interest and there exists an amino acid residue having a frequency of appearance of 60% or more, an amino acid residue having the highest frequency of appearance is identified as a consensus residue, and when there exist two or more amino acid residues having the highest frequency of appearance, an amino acid residue having the smallest molecular weight among the amino acid residues is identified as a consensus residue, and
   (i-v) if none of the above (i-i) to (i-iv) is appropriate, the amino acid residue of the GPCR of interest is identified as a consensus residue;
(ii) when the consensus residues are identified in accordance with the criterion (i) and when a consensus residue nearest to the N terminus is a consensus residue at a position corresponding to the N terminus of the GPCR of interest or a C-terminal side thereof and is not a methionine residue, a consensus residue on an N-terminal side of a consensus residue consisting of a methionine residue positioned nearest to the N terminus is changed to the absence of a consensus residue; and
(iii) when the consensus residues are identified in accordance with the criterion (i) and when a consensus residue nearest to the N terminus is a consensus residue at a position corresponding to an N-terminal side of the N terminus of the GPCR of interest and is not a methionine residue, an amino acid residue having the highest frequency of appearance is identified as a consensus residue by tracing back one by one amino acid positions on an N-terminal side of the position of the consensus residue of the alignment until a methionine residue appears, and when there exist two or more amino acid residues having the highest frequency of appearance, an amino acid residue having the smallest molecular weight among the amino acid residues is identified as a consensus residue.

In this context, the "frequency of appearance" refers to a percentage of the number of particular amino acid residues appearing at each amino acid position in the alignment of amino acid sequences with respect to the number of amino acid sequences subjected to the alignment. The alignment of amino acid sequences can be carried out with an algorithm known in the art.

The criterion (i), (i-i) is a criterion in the case where there exists an amino acid residue in the GPCR of interest at an amino acid position in the alignment of the amino acid sequence of the GPCR of interest and the amino acid sequences of GPCRs encoded by orthologs of the GPCR of interest in vertebrates. In this respect, provided that there exists one amino acid residue which is different from the amino acid residue of the GPCR of interest and has a frequency of appearance of 50% or more, the amino acid residue having a frequency of appearance of 50% or more is identified as a consensus residue at the position. On the other hand, provided that all amino acid residues other than the amino acid residue of the GPCR of interest have a frequency of appearance of less than 50%, the amino acid residue of the GPCR of interest is identified as a consensus residue at the position in accordance with the criterion (i-v).

The criterion (i), (i-ii) is a criterion in the case where there exists an amino acid residue in the GPCR of interest at an amino acid position in the alignment of the amino acid sequence of the GPCR of interest and the amino acid sequences of GPCRs encoded by orthologs of the GPCR of interest in vertebrates. In this respect, provided that there exist two amino acid residues having a frequency of appearance of 50%, one of the two amino acid residues is inevitably the amino acid residue of the GPCR of interest and the amino acid residue of the GPCR of interest is identified as a consensus residue at the position.

The criterion (i), (i-iii) is a criterion in the case where there exists an amino acid residue in the GPCR of interest at an amino acid position in the alignment of the amino acid sequence of the GPCR of interest and the amino acid sequences of GPCRs encoded by orthologs of the GPCR of interest in vertebrates. In this respect, provided that there exists no amino acid residue having a frequency of appearance of 40% or more, the absence of a consensus residue is identified at the position. On the other hand, provided that the frequency of appearance in the absence of an amino acid residue is less than 40%, a consensus residue at the position is identified in accordance with the criterion (i-i) when the criterion (i-i) is appropriate, while the amino acid residue of the GPCR of interest is identified as a consensus residue at the position in accordance with the criterion (i-v) when the criterion (i-i) is not appropriate.

The criterion (i), (i-iv) is a criterion in the case where there exists no amino acid residue in the GPCR of interest at an amino acid position in the alignment of the amino acid sequence of the GPCR of interest and the amino acid sequences of GPCRs encoded by orthologs of the GPCR of interest in vertebrates. In this respect, provided that there exists an amino acid residue having a frequency of appearance of 60% or more, an amino acid residue having the highest frequency of appearance is identified as a consensus residue at the position, and when there exist two or more amino acid residues having the highest frequency of appearance, an amino acid residue having the smallest molecular weight among the amino acids having the highest frequency of appearance is identified as a consensus residue. For example, provided that one amino acid residue has the highest frequency of appearance, the one amino acid residue is identified as a consensus residue at the position. Provided that two or more amino acid residues have the highest frequency of appearance, an amino acid residue having the smallest molecular weight thereamong can be identified as a consensus residue at the position. If the change extends the full-length of the consensus amino acid sequence by 10% or more on an N-terminal side from the full-length of the amino acid sequence of the GPCR of interest, a consensus residue at a position corresponding to the N terminus of the GPCR of interest may be set to a methionine residue and a consensus residue on an N-terminal side of the methionine residue may be changed to the absence, in the consensus amino acid sequence from the viewpoint of structural maintenance of the GPCR. Specifically, an N-terminal structure of the GPCR of interest may be maintained as it is. On the other hand, provided that the frequency of appearance in the presence of an amino acid residue is less than 60%, the amino acid residue of the GPCR of interest is identified as a consensus residue at the position in accordance with the criterion (i-v). Specifically, the absence of an amino acid residue is identified at the position.

If none of the above (i), (i-i) to (i-iv) is appropriate for an amino acid position in the alignment of the amino acid sequence of the GPCR of interest and the amino acid sequences of GPCRs encoded by orthologs of the GPCR of interest in vertebrates, the amino acid residue of the GPCR of interest is identified as a consensus residue at the position. In this respect, provided that there exists no amino acid residue in the GPCR of interest, the absence of an amino acid residue can be identified at the position of the consensus amino acid sequence.

The criterion (ii) is a criterion in the case where when the consensus residue is identified at each amino acid position in the alignment of the amino acid sequence of the GPCR of interest and the amino acid sequences of GPCRs encoded by orthologs of the GPCR of interest in vertebrates in accordance with the criterion (i), a consensus residue positioned nearest to the N terminus among the consensus residues is a consensus residue at a position corresponding to the N terminus of the GPCR of interest or a C-terminal side thereof and is not a methionine residue. In this respect, a consensus residue on an N-terminal side of a consensus residue consisting of a methionine residue positioned nearest to the N terminus among the consensus residues is changed to the absence of a consensus residue such that an N-terminal consensus residue is a methionine residue, in other words, such that a translation initiation amino acid of the GPCR polypeptide is a methionine residue. For example, the consensus residues are checked one by one from the N terminus, and the absence of a consensus residue is identified at a position having no methionine residue. This operation can be repeated until a methionine residue appears for the first time. If the change decreases the full-length of the consensus amino acid sequence by 10% or more from the full-length of the amino acid sequence of the GPCR of interest, a consensus residue nearest to the N terminus among the consensus residues before the change may be changed to a consensus residue consisting of a methionine residue from the viewpoint of structural maintenance of the helix of the GPCR. In this respect, provided that the consensus residue nearest to the N terminus among the consensus residues before the change is asparagine, serine, or threonine involved in sugar chain modification and/or membrane translocation, an amino acid residue of the GPCR of interest on an N-terminal side of a position corresponding to the consensus residue may be used as a consensus residue without changing the consensus residue nearest to the N terminus among the consensus residues before the change from the viewpoint of structural maintenance of the GPCR. Specifically, an N-terminal structure of the GPCR of interest may be maintained as it is.

The criterion (iii) is a criterion in the case where when the consensus residue is identified at each amino acid position in the alignment of the amino acid sequence of the GPCR of interest and the amino acid sequences of GPCRs encoded by orthologs of the GPCR of interest in vertebrates in accordance with the criterion (i), a consensus residue nearest to the N terminus is a consensus residue at a position corresponding to an N-terminal side of the N terminus of the GPCR of interest and is not a methionine residue. In this respect, the alignment is checked by tracing back one by one amino acid positions on an N-terminal side of the position of a consensus residue nearest to the N terminus until a methionine residue appears such that an N-terminal consensus residue is a methionine residue, in other words, such that a translation initiation amino acid of the GPCR polypeptide is a methionine residue. An amino acid residue having the highest frequency of appearance is identified as a consensus residue. When there exist two or more amino acid residues having the highest frequency of appearance, an amino acid residue having the smallest molecular weight among the amino acid residues is identified as a consensus residue.

An amino acid sequence consisting of the consensus residues thus identified is the consensus amino acid sequence. The GPCR polypeptide used in the present invention is an altered GPCR polypeptide consisting of an amino acid sequence obtained by, in an amino acid sequence of an GPCR of interest, altering at least one amino acid residue different from that in a consensus amino acid sequence to an amino acid residue at a position corresponding thereto in the consensus amino acid sequence. In this context, the term "altering" conceptually includes all of substitution, deletion, addition, and insertion. The difference between the amino acid sequence of the GPCR of interest and the consensus amino acid sequence can be found, for example, by aligning the amino acid sequences and comparing both the amino acid sequences using an algorithm known in the art. For example, provided that an amino acid residue at an amino acid position in the amino acid sequence of the GPCR of interest is an amino acid residue different from an amino acid residue at a position corresponding thereto in the consensus amino acid sequence when the amino acid sequence of the GPCR of interest is compared with the consensus amino acid sequence, the amino acid residue of the GPCR of interest can be substituted by the amino acid residue of the consensus amino acid sequence. Alternatively, provided that an amino acid residue at a position corresponding to an amino acid position in the amino acid sequence of the GPCR of interest is absent in the consensus amino acid sequence when the amino acid sequence of the GPCR of interest is compared with the consensus amino acid sequence, an amino acid residue at the amino acid position of the GPCR of interest can be deleted. Alternatively, provided that an amino acid residue at a position corresponding to a position having no amino acid residue in the amino acid sequence of the GPCR of interest is present in the consensus amino acid sequence when the amino acid sequence of the GPCR of interest is compared with the consensus amino acid sequence, the amino acid residue of the consensus amino acid sequence can be inserted to the amino acid position of the GPCR of interest. Provided that the consensus amino acid sequence is longer at the N terminus than the amino acid sequence of the GPCR of interest when the amino acid sequence of the GPCR of interest is compared with the consensus amino acid sequence, an N-terminal moiety present only in the consensus amino acid sequence can be added to the N terminus of the amino acid sequence of the GPCR of interest. Alternatively, provided that the consensus amino acid sequence is longer at its C terminus than the amino acid sequence of the GPCR of interest when the amino acid sequence of the GPCR of interest is compared with the consensus amino acid sequence, a C-terminal moiety present only in the consensus amino acid sequence can be added to the C terminus of the amino acid sequence of the GPCR of interest.

The number of amino acid residues to be altered in the amino acid sequence of the GPCR of interest is at least 1, preferably at least 5, more preferably at least 10. Further more preferably, all amino acid residues different from those of the consensus amino acid sequence in the amino acid sequence of the GPCR of interest are altered to the amino acid residues at positions corresponding thereto in the consensus amino acid sequence (i.e., the amino acid sequence of the GPCR of interest is altered to the consensus amino acid sequence).

Alternatively, the number of amino acid residues to be altered in the amino acid sequence of the GPCR of interest can be the number of preferably at least 10%, more preferably at least 30%, further more preferably at least 50%, further more preferably at least 70%, further more preferably at least 90%, further more preferably 100% in the number of amino acid residues different from those of the consensus amino acid sequence (i.e., the amino acid sequence of the GPCR of interest is altered to the consensus amino acid sequence).

Provided that the consensus amino acid sequence is longer at the N terminus than the amino acid sequence of the GPCR of interest, it is preferred to perform the alteration by integrally adding an N-terminal moiety present only in the consensus amino acid sequence to the N terminus of the amino acid sequence of the GPCR of interest, irrespective of the number of amino acid residues. Provided that the consensus amino acid sequence is shorter at the N terminus than the amino acid sequence of the GPCR of interest, it is preferred to perform the alteration by integrally deleting an N-terminal moiety present only in the amino acid sequence of the GPCR of interest from the N terminus of the amino acid sequence of the GPCR of interest, irrespective of the number of amino acid residues. Provided that the consensus amino acid sequence is longer at the C terminus than the amino acid sequence of the GPCR of interest, it is preferred to perform the alteration by integrally adding a C-terminal moiety present only in the consensus amino acid sequence to the C terminus of the amino acid sequence of the GPCR of interest, irrespective of the number of amino acid residues. Provided that the consensus amino acid sequence is shorter at the C terminus than the amino acid sequence of the GPCR of interest, it is preferred to perform the alteration by integrally deleting a C-terminal moiety present only in the amino acid sequence of the GPCR of interest from the C terminus of the amino acid sequence of the GPCR of interest, irrespective of the number of amino acid residues.

As the GPCR polypeptide used in the present invention, a polypeptide containing substitution, deletion, addition, or insertion of one to several (e.g., 1 to 10, 1 to 5, or 1 to 3) amino acid residues at amino acid positions other than the amino acid position to be altered on the basis of the consensus amino acid sequence described above is also included in the present invention without impairing its functions.

In a preferred example of the present embodiment, the GPCR polypeptide consists of an amino acid sequence obtained by, in an amino acid sequence of sequence identification number (2) of an GPCR (1) in Tables 1-1 and 1-2 given below, altering at least one amino acid residue different from that in a consensus amino acid sequence of sequence identification number (3) to the amino acid residue at a position corresponding thereto in the consensus amino acid sequence. More preferably, the GPCR polypeptide is a GPCR polypeptide consisting of an amino acid sequence of any of SEQ ID Nos: 108 to 214 and 275 to 312 in (3) of Table 1-1 and 1-2 given below, or a combination of a GPCR polypeptide consisting of an amino acid sequence of SEQ ID NO: 112 or 113 and a GPCR polypeptide consisting of the amino acid sequence of SEQ ID NO: 114 in (3) of Table 1-1 given below. The GPCR polypeptide consisting of an amino acid sequence of any of SEQ ID NOs: 108 to 112, 114 to 120, 138 to 145, 167, 183, 187, 192, and 209 is highly improved in membrane expression in comparison with the original GPCR. In Tables 1-1 and 1-2, the GPCRs (1) of Nos. 1 to 145 are human GPCRs, and Accession No. represents GenBank Accession No.

**[Table 1-1]**

| No. | (1) GPCR | Accession No. | (2) SEQ ID NO of amino acid sequence | (3) SEQ ID NO of consensus amino acid sequence | No. | (1) GPCR | Accession No. | (2) SEQ ID NO of amino acid sequence | (3) SEQ ID NO of consensus amino acid sequence |
|---|---|---|---|---|---|---|---|---|---|
| 1 | VN1R1 | NP_065684.1 | 1 | 108 | 55 | GPR21 | NP_005285.1 | 55 | 162 |
| 2 | VN1R2 | NP_776255.2 | 2 | 109 | 56 | GPR22 | NP_005286.2 | 56 | 163 |
| 3 | VN1R4 | NP_776256.2 | 3 | 110 | 57 | GPR25 | NP_005289.2 | 57 | 164 |
| 4 | VN1R5 | NP_776257.1 | 4 | 111 | 58 | GPR26 | NP_703143.1 | 58 | 165 |
| 5 | TAS1R1 | NP_619642.2 | 5 | 112 | 59 | GPR27 | NP_061844.1 | 59 | 166 |
| 6 | TAS1R2 | NP_689418.2 | 6 | 113 | 60 | GPR31 | NP_005290.2 | 60 | 167 |
| 7 | TAS1R3 | NP_689414.2 | 7 | 114 | 61 | GPR32 | NP_001497.1 | 61 | 168 |
| 8 | TAS2R8 | NP_076407.1 | 8 | 115 | 62 | GPR33 | NP_001184113.2 | 62 | 169 |
| 9 | TAS2R16 | NP_058641.1 | 9 | 116 | 63 | GPR34 | NP_001091048.1 | 63 | 170 |
| 10 | TAS2R38 | NP_789787.5 | 10 | 117 | 64 | GPR35 | NP_001182310.1 | 64 | 171 |
| 11 | TAS2R42 | NP_852094.2 | 11 | 118 | 65 | GPR37 | NP_005293.1 | 65 | 172 |
| 12 | TAS2R45 | NP_795367.2 | 12 | 119 | 66 | GPR37L1 | NP_004758.3 | 66 | 173 |
| 13 | TAS2R46 | NP_795368.2 | 13 | 120 | 67 | GPR39 | NP_001499.1 | 67 | 174 |
| 14 | TAS2R31 | NP_795366.2 | 14 | 121 | 68 | GPR45 | NP_009158.3 | 68 | 175 |
| 15 | TAS2R1 | NP_001373277.1 | 15 | 122 | 69 | GPR48 | NP_001333361.1 | 69 | 176 |
| 16 | TAS2R3 | NP_058639.1 | 16 | 123 | 70 | GPR49 | NP_003658.1 | 70 | 177 |
| 17 | TAS2R4 | NP_058640.1 | 17 | 124 | 71 | GPR50 | NP_004215.2 | 71 | 178 |
| 18 | TAS2R5 | NP_061853.1 | 18 | 125 | 72 | GPR52 | NP_005675.3 | 72 | 179 |
| 19 | TAS2R7 | NP_076408.1 | 19 | 126 | 73 | GPR61 | NP_001380836.1 | 73 | 180 |
| 20 | TAS2R9 | NP_076406.1 | 20 | 127 | 74 | GPR62 | NP_543141.3 | 74 | 181 |
| 21 | TAS2R10 | NP_076410.1 | 21 | 128 | 75 | GPR63 | NP_001137429.1 | 75 | 182 |
| 22 | TAS2R13 | NP_076409.1 | 22 | 129 | 76 | GPR65 | NP_003599.2 | 76 | 183 |
| 23 | TAS2R14 | NP_076411.1 | 23 | 130 | 77 | GPR68 | XP_005268167.1 | 77 | 184 |
| 24 | TAS2R20 | NP_795370.2 | 24 | 131 | 78 | GPR75 | NP_006785.1 | 78 | 185 |
| 25 | TAS2R30 | NP_001091112.1 | 25 | 132 | 79 | GPR78 | NP_543009.2 | 79 | 186 |
| 26 | TAS2R39 | NP_795362.2 | 26 | 133 | 80 | GPR82 | NP 543007.1 | 80 | 187 |
| 27 | TAS2R40 | NP_795363.1 | 27 | 134 | 81 | GPR83 | NP_001317274.1 | 81 | 188 |
| 28 | TAS2R43 | NP_795365.2 | 28 | 135 | 82 | GPR84 | NP_065103.1 | 82 | 189 |
| 29 | TAS2R50 | NP_795371.2 | 29 | 136 | 83 | GPR85 | NP_001139737.1 | 83 | 190 |
| 30 | TAS2R60 | NP_803186.1 | 30 | 137 | 84 | GPR87 | NP_076404.3 | 84 | 191 |
| 31 | TAAR1 | NP_612200.1 | 31 | 138 | 85 | GPR88 | NP_071332.2 | 85 | 192 |
| 32 | TAAR2 | NP_001028252.1 | 32 | 139 | 86 | GPR101 | NP_473362.1 | 86 | 193 |
| 33 | TAAR5 | NP_003958.2 | 33 | 140 | 87 | GPR132 | NP_001265623.1 | 87 | 194 |
| 34 | TAAR8 | NP_444508.1 | 34 | 141 | 88 | GPR135 | NP_072093.2 | 88 | 195 |
| 35 | TAAR9 | NP_778227.3 | 35 | 142 | 89 | GPR139 | NP_001002911.1 | 89 | 196 |
| 36 | TAAR6 | NP_778237.1 | 36 | 143 | 90 | GPR141 | NP_001316922.1 | 90 | 197 |
| 37 | MrgprE | NP_001034254.2 | 37 | 144 | 91 | GPR142 | NP_001318005.1 | 91 | 198 |
| 38 | MrgprF | NP_001091985.1 | 38 | 145 | 92 | GPR146 | NP_001290402.1 | 92 | 199 |
| 39 | MAS1 | NP_001353633.1 | 39 | 146 | 93 | GPR148 | NP_997247.2 | 93 | 200 |
| 40 | MAS1L | NP_443199.1 | 40 | 147 | 94 | GPR149 | NP_001033794.1 | 94 | 201 |
| 41 | MRGPRD | NP_944605.2 | 41 | 148 | 95 | GPR150 | NP_954713.1 | 95 | 202 |
| 42 | MRGPRG | NP_001157849.1 | 42 | 149 | 96 | GPR151 | NP_919227.2 | 96 | 203 |
| 43 | MRGPRX1 | NP_001380507.1 | 43 | 150 | 97 | GPR152 | NP_996880.1 | 97 | 204 |
| 44 | MRGPRX2 | NP_001290544.1 | 44 | 151 | 98 | GPR153 | NP_997253.2 | 98 | 205 |
| 45 | MRGPRX3 | NP_001357393.1 | 45 | 152 | 99 | GPR160 | NP_055188.1 | 99 | 206 |
| 46 | MRGPRX4 | NP_473373.2 | 46 | 153 | 100 | GPR161 | NP_001254538.1 | 100 | 207 |
| 47 | GPR3 | NP_005272.1 | 47 | 154 | 101 | GPR162 | NP_055264.1 | 101 | 208 |
| 48 | GPR4 | NP_005273.1 | 48 | 155 | 102 | GPR171 | NP_037440.3 | 102 | 209 |
| 49 | GPR6 | NP_601273028.1 | 49 | 156 | 103 | GPR173 | NP_061842.1 | 103 | 210 |
| 50 | GPR12 | NP_005279.1 | 50 | 157 | 104 | GPR174 | NP_115942.1 | 104 | 211 |
| 51 | GPR15 | NP_005281.1 | 51 | 158 | 105 | GPR176 | NP_001258783.1 | 105 | 212 |
| 52 | GPR17 | NP_001154887.1 | 52 | 159 | 106 | GPR182 | NP_009195.1 | 106 | 213 |
| 53 | GPR19 | NP_006134.2 | 53 | 160 | 107 | GPR183 | NP_004942.1 | 107 | 214 |
| 54 | GPR20 | NP_005284.2 | 54 | 161 | | | | | |

**[Table 1-2]**

| No. | (1) GPCR | Accession No. | (2) SEQ ID NO of amino acid sequence | (3) SEQ ID NO of consensus amino acid sequence | No. | (1) GPCR | Accession No. | (2) SEQ ID NO of amino acid sequence | (3) SEQ ID NO of consensus amino acid sequence |
|---|---|---|---|---|---|---|---|---|---|
| 108 | GPR42 | NP_001335124.1 | 237 | 275 | 127 | ADGRF5 | NP_001091988.1 | 256 | 294 |
| 109 | ADGRA1 | NP_001077378.1 | 238 | 276 | 128 | ADGRG1 | NP_001357368.1 | 257 | 295 |
| 110 | ADGRA2 | NP_116166.9 | 239 | 277 | 129 | ADGRG2 | NP_001073327.1 | 258 | 296 |
| 111 | ADGRA3 | XP_005248194.1 | 240 | 278 | 130 | ADGRG4 | NP_722576.3 | 259 | 297 |
| 112 | ADGRB1 | NP_001693.2 | 241 | 279 | 131 | ADGRG5 | NP_001291305.1 | 260 | 298 |
| 113 | ADGRB2 | NP_001281264.1 | 242 | 280 | 132 | ADGRG6 | NP_001027566.2 | 261 | 299 |
| 114 | ADGRB3 | NP_001695.2 | 243 | 281 | 133 | ADGRG7 | NP_001295291.1 | 262 | 300 |
| 115 | ADGRC1 | NP_001365257.1 | 244 | 282 | 134 | ADGRL1 | NP_001008701.1 | 263 | 301 |
| 116 | ADGRC2 | NP_001399.1 | 245 | 283 | 135 | ADGRL2 | NP_001284633.1 | 264 | 302 |
| 117 | ADGRC3 | NP_001398 | 246 | 284 | 136 | ADGRL3 | NP_001309175.1 | 265 | 303 |
| 118 | ADGRD1 | NP_001317426.1 | 247 | 285 | 137 | ADGRL4 | NP_071442.2 | 266 | 304 |
| 119 | ADGRD2 | NP_001382354.1 | 248 | 286 | 138 | Chrm-4/M4R | NP_000732.2 | 267 | 305 |
| 120 | ADGRE1 | NP_001243181.1 | 249 | 287 | 139 | F2RL1 | NP_005233.4 | 268 | 306 |
| 121 | ADGRE2 | NP_001257981.1 | 250 | 288 | 140 | GRM5 | NP_001137303.1 | 269 | 307 |
| 122 | ADGRE3 | NP_001276087.1 | 251 | 289 | 141 | APLNR | NP_005152.1 | 270 | 308 |
| 123 | ADGRE5 | NP_001020331.1 | 252 | 290 | 142 | CALCRL | NP_001258680.1 | 271 | 309 |
| 124 | ADGRF1 | NP_722582.2 | 253 | 291 | 143 | GLP2R | NP_004237.1 | 272 | 310 |
| 125 | ADGRF3 | NP_001138640.1 | 254 | 292 | 144 | MC4R | NP_005903.2 | 273 | 311 |
| 126 | ADGRF4 | NP_001334784.1 | 255 | 293 | 145 | CCR6 | NP_001381511.1 | 274 | 312 |

The GPCR polypeptides consisting of an amino acid sequence obtained by, in an amino acid sequence of sequence identification number (2) of any of the GPCRs (1) of Nos. 1 to 4 in the above Table 1-1, altering at least one amino acid residue different from that in a consensus amino acid sequence of sequence identification number (3) to an amino acid residue at a position corresponding thereto in the consensus amino acid sequence are vomeronasal receptor polypeptides. In the above Table 1-1, the vomeronasal receptor polypeptide consisting of an amino acid sequence of any of SEQ ID NOs: 108 to 111 in (3) is a consensus vomeronasal receptor consisting of an amino acid sequence of any of SEQ ID NOs: 1 to 4 in (2) of the vomeronasal receptors (1) of Nos. 1 to 4, and a consensus amino acid sequence derived from an amino acid sequence of a vomeronasal receptor encoded by orthologs of the vomeronasal receptor in vertebrates.

The GPCR polypeptides consisting of an amino acid sequence obtained by, in an amino acid sequence of sequence identification number (2) of any of the GPCRs (1) of Nos. 5 to 30 in the above Table 1-1, altering at least one amino acid residue different from that in a consensus amino acid sequence of sequence identification number (3) to an amino acid residue at a position corresponding thereto in the consensus amino acid sequence are taste receptor polypeptides. Among these, the GPCR polypeptide consisting of an amino acid sequence obtained by, in the amino acid sequence of sequence identification number (2) of the GPCR (1) of No. 5, altering at least one amino acid residue different from that in a consensus amino acid sequence of sequence identification number (3) to an amino acid residue at a position corresponding thereto in the consensus amino acid sequence, and the GPCR polypeptide consisting of an amino acid sequence obtained by, in the amino acid sequence of sequence identification number (2) of the GPCR (1) of No. 7, altering at least one amino acid residue different from that in a consensus amino acid sequence of sequence identification number (3) to the amino acid residue at a position corresponding thereto in the consensus amino acid sequence form an umami receptor complex. In addition, the GPCR polypeptide consisting of an amino acid sequence obtained by, in the amino acid sequence of sequence identification number (2) of the GPCR (1) of No. 6, altering at least one amino acid residue different from that in a consensus amino acid sequence of sequence identification number (3) to an amino acid residue at a position corresponding thereto in the consensus amino acid sequence, and the GPCR polypeptide consisting of an amino acid sequence obtained by, in the amino acid sequence of sequence identification number (2) of the GPCR (1) of No. 7, altering at least one amino acid residue different from that in a consensus amino acid sequence of sequence identification number (3) to the amino acid residue at a position corresponding thereto in the consensus amino acid sequence form a sweetness receptor complex. On the other hand, the GPCR polypeptides consisting of an amino acid sequence obtained by, in an amino acid sequence of sequence identification number (2) of any of the GPCRs (1) of Nos. 8 to 30, altering at least one amino acid residue different from that in a consensus amino acid sequence of sequence identification number (3) to an amino acid residue at a position corresponding thereto in the consensus amino acid sequence are bitterness receptor polypeptides. In the above Table 1-1, the taste receptor polypeptide consisting of an amino acid sequence of any of SEQ ID NOs: 112 to 137 in (3) is a consensus taste receptor consisting of an amino acid sequence of any of SEQ ID NOs: 5 to 30 in (2) of the taste receptors (1) of Nos. 5 to 30, and a consensus amino acid sequence derived from the amino acid sequence of a taste receptor encoded by orthologs of the taste receptor in vertebrates.

The GPCR polypeptides consisting of an amino acid sequence obtained by, in an amino acid sequence of sequence identification number (2) of any of the GPCRs (1) of Nos. 31 to 36 in the above Table 1-1, altering at least one amino acid residue different from that in a consensus amino acid sequence of sequence identification number (3) to an amino acid residue at a position corresponding thereto in the consensus amino acid sequence are trace amine-associated receptor polypeptides. In the above Table 1-1, the trace amine-associated receptor polypeptide consisting of an amino acid sequence of any of SEQ ID NOs: 138 to 143 in (3) is a consensus trace amine-associated receptor polypeptide consisting of an amino acid sequence of any of SEQ ID NOs: 31 to 36 in (2) of the trace amine-associated receptor polypeptides (1) of Nos. 31 to 36, and a consensus amino acid sequence derived from an amino acid sequence of a trace amine-associated receptor polypeptide encoded by orthologs of the trace amine-associated receptor polypeptide in vertebrates. Among these, the trace amine-associated receptor polypeptide consisting of the amino acid sequence of SEQ ID NO: 143 is a consensus trace amine-associated receptor consisting of a consensus amino acid sequence obtained by maintaining an N-terminal structure of the trace amine-associated receptor polypeptide of interest as it is in the criterion (ii).

The GPCR polypeptides consisting of an amino acid sequence obtained by, in an amino acid sequence of sequence identification number (2) of any of the GPCRs (1) of Nos. 37 to 46 in the above Table 1-1, altering at least one amino acid residue different from that in a consensus amino acid sequence of sequence identification number (3) to an amino acid residue at a position corresponding thereto of the consensus amino acid sequence are Mas-related G protein-coupled receptor polypeptides. In the above Table 1-1, the Mas-related G protein-coupled receptor polypeptide consisting of an amino acid sequence of any of SEQ ID NOs: 144 to 153 in (3) is a consensus Mas-related G protein-coupled receptor consisting of an amino acid sequence of any of SEQ ID NOs: 37 to 46 in (2) of the GPCRs (1) of Nos. 37 to 46, and a consensus amino acid sequence derived from an amino acid sequence of a Mas-related G protein-coupled receptor encoded by orthologs of the Mas-related G protein-coupled receptor in vertebrates.

The GPCR polypeptides consisting of an amino acid sequence obtained by, in an amino acid sequence of sequence identification number (2) of any of the GPCRs (1) of Nos. 47 to 108 in the above Tables 1-1 and 1-2, altering at least one amino acid residue different from that in a consensus amino acid sequence of sequence identification number (3) to an amino acid residue at a position corresponding thereto in the consensus amino acid sequence are GPR polypeptides. In the above Tables 1-1 and 1-2, the GPR polypeptide consisting of an amino acid sequence of any of SEQ ID NOs: 154 to 214 and 275 in (3) is a consensus GPR consisting of an amino acid sequence of any of SEQ ID NOs: 47 to 107 and 237 in (2) of the GPRs (1) of Nos. 47 to 108, and a consensus amino acid sequence derived from an amino acid sequence of a GPR encoded by orthologs of the GPR in vertebrates.

The GPCR polypeptides consisting of an amino acid sequence obtained by, in an amino acid sequence of sequence identification number (2) of any of the GPCRs (1) of Nos. 109 to 145 in the above Table 1-2, altering at least one amino acid residue different from that in a consensus amino acid sequence of sequence identification number (3) to an amino acid residue at a position corresponding thereto in the consensus amino acid sequence are GPCR polypeptides other than vomeronasal receptor polypeptides, taste receptor polypeptides, trace amine-associated receptor polypeptides, Mas-related G protein-coupled receptor polypeptides, GPR polypeptides, and olfactory receptor polypeptides. In the above Table 1-2, the GPCR polypeptide consisting of an amino acid sequence of any of SEQ ID NOs: 276 to 312 in (3) is a consensus GPCR consisting of an amino acid sequence of any of SEQ ID NOs: 238 to 274 in (2) of the GPCRs (1) of Nos. 109 to 145, and a consensus amino acid sequence derived from an amino acid sequence of a GPCR encoded by orthologs of the GPCR in vertebrates.

The "expression" of the GPCR polypeptide of the present invention means that a translation product is produced from a polynucleotide encoding the polypeptide and the translation product is localized in a functional state in a cell membrane which is a site of its action. The GPCR polypeptide of the present invention can be expressed in cells by an approach known in the art. For example, the GPCR polypeptide can be expressed on cell membranes of cells by transferring a vector containing a polynucleotide encoding the polypeptide to the cells serving as a host, or by transferring a DNA fragment containing a polynucleotide encoding the polypeptide to the genome of the cells serving as a host. Preferably, the GPCR polypeptide is expressed on cell membranes of host cells by transforming the host cells using a vector containing a polynucleotide encoding the polypeptide. The host cells may not be limited as long as the cells can functionally express a foreign GPCR. Specific examples of the cells include, but are not limited to, human embryonic kidney cells (HEK293 cells), Chinese hamster cells (CHO cells), monkey cells (COS cells), isolated olfactory neurons, *Xenopus* oocytes, insect cells, yeasts, and bacteria.

The polynucleotide encoding the GPCR polypeptide can be obtained by use of various mutagenesis techniques known in the art. The polynucleotide encoding the GPCR polypeptide can be obtained, for example, by altering a nucleotide sequence encoding the amino acid residue to be altered to a nucleotide sequence encoding an amino acid residue after alteration in a polynucleotide encoding the amino acid sequence of the GPCR of interest.

A mutation of interest can be introduced to the polynucleotide encoding the amino acid sequence of the GPCR of interest by use of various site-directed mutagenesis techniques well known to those skilled in the art. The site-directed mutagenesis techniques can be performed by an arbitrary approach, for example, inverse PCR or annealing. A commercially available kit for site-directed mutagenesis (e.g., QuickChange II Site-Directed Mutagenesis Kit or QuickChange Multi Site-Directed Mutagenesis Kit from Stratagene California) may be used.

Alternatively, the polynucleotide encoding the GPCR polypeptide can also be obtained by genome editing using artificial DNA nucleases or programmable nuclease, DNA synthesis based on the nucleotide sequence, or the like.

The polynucleotide encoding the GPCR polypeptide can contain single-stranded or double-stranded DNA, cDNA, RNA, or other artificial nucleic acids. The DNA, the cDNA, and the RNA may be chemically synthesized. The polynucleotide may contain nucleotide sequences of an open reading frame (ORF) as well as an untranslated region (UTR). The polynucleotide may be codon-optimized for the species of cells for GPCR expressions. Information on codons for use in various organisms is available from Codon Usage Database ([www.kazusa.or.jp/codon/]) .

In a preferred example, the polynucleotide encoding the GPCR polypeptide consists of a nucleotide sequence of any of SEQ ID NOs: 215 to 234 and 313 to 317. These polynucleotides encode GPCR polypeptides consisting of amino acid sequences of SEQ ID NOs: 108 to 112, 114 to 120, 138 to 145, 167, 183, 187, 192, and 209, respectively.

The obtained polynucleotide encoding the GPCR polypeptide can be incorporated into a vector or a DNA fragment. Preferably, the vector is an expression vector. Also preferably, the vector is an expression vector which can transfer the GPCR polynucleotide to host cells and enables the polynucleotide to be expressed in the host cells. Preferably, the vector may be a vector, such as a plasmid, capable of autonomously proliferating or replicating extrachromosomally, or may be a vector which is integrated into the chromosome. Specific examples of the vector include, but are not limited to, pME18S.

The vector preferably contains the polynucleotide encoding the GPCR polypeptide, and a control region operably linked thereto. The control region is a sequence for expressing the transferred polynucleotide encoding the GPCR polypeptide in host cells harboring the vector. Examples thereof include expression regulatory regions such as promoters and terminators, and transcription initiation points. The type of the control region can be appropriately selected depending on the type of the vector. The vector or the DNA fragment may optionally further contain a drug (e.g., ampicillin) resistance gene as a selective marker. The control region and the selective marker gene originally contained in the vector may be used, or the control region and the selective marker gene may be incorporated, together with or separately from the polynucleotide encoding the GPCR polypeptide, to the vector.

Examples of the DNA fragment containing the polynucleotide encoding the GPCR polypeptide include PCR-amplified DNA fragments and restriction enzyme-cleaved DNA fragments. Preferably, the DNA fragment can be an expression cassette containing the polynucleotide and a control region operably linked thereto. Examples of the control region that can be used are the same as in the case of the vector.

Preferably, a polynucleotide encoding RTP (receptortransporting protein) (in the present specification, this polynucleotide is also referred to as RTP gene) is transferred, together with the polynucleotide encoding the GPCR polypeptide, into cells in order to promote the cell membrane expression of the GPCR polypeptide. Alternatively, preferably, a polynucleotide encoding REEP (receptor expression enhancing protein) (in the present specification, this polynucleotide is also referred to as REEP gene) is transferred, together with the polynucleotide encoding the GPCR polypeptide, into cells in order to promote the cell membrane expression of the GPCR polypeptide. For example, a vector containing the RTP gene or REEP gene and the polynucleotide encoding the GPCR polypeptide may be constructed and transferred into host cells, or a vector containing the RTP gene or REEP gene and a vector containing the polynucleotide encoding the GPCR polypeptide may be transferred into host cells. Examples of the RTP include RTP1S, RTP3, and RTP4, and examples of the RTP1S include human RTP1S. The human RTP1S is registered as AY562235 in GenBank and is a polypeptide which consists of the amino acid sequence of SEQ ID NOs: 236 and is encoded by a gene having the nucleotide sequence of SEQ ID NOs: 235. Examples of the REEP include REEP1 and REEP2, examples of the REEP1 include human REEP1, and examples of the REEP2 include human REEP2.

A general transformation technique for mammalian cells, for example, an electroporation technique, a lipofection technique, or a particle gun technique, can be used in the transfer of the vector or the DNA fragment into host cells. Transformed cells harboring the vector or the DNA fragment of interest can be selected by exploiting the selective marker. Alternatively, the transfer of the vector or the DNA fragment of interest may be confirmed by examining the DNA sequences of the cells.

The GPCR polypeptide of the present invention is produced from the polynucleotide encoding the GPCR polypeptide contained in the vector or the DNA fragment transferred into cells by the procedures described above, and integrated to cell membranes. Accordingly, the GPCR polypeptide expressed by the expression method of the present invention is expressed in cell membranes of transformed cells genetically manipulated so as to express the GPCR polypeptide.

The cell membrane expression (level) of the GPCR polypeptide of the present invention can be measured, for example, by fusing a tag such as a FLAG tag with the GPCR polypeptide in advance, and using an antibody which specifically recognizes the tag in an approach known in the art such as flow cytometry.

The GPCR polypeptide expressed by the expression method of the present invention can be expressed on a cultured cell membrane more efficiently in comparison with the original GPCR. In addition, a response of the GPCR polypeptide is considered to reflect a response of the original GPCR. Thus, in an alternative aspect, the present invention provides a method for measuring a response of a GPCR of interest. The method includes measuring a response of the GPCR polypeptide expressed by the method for expressing a GPCR polypeptide according to the present invention. The response measurement method of the present invention can improve the response measurement efficiency of a GPCR of interest and enables response measurement of a GPCR of interest never before possible to functionally analyze due to insufficient cell membrane expression in cultured cells.

The GPCR polypeptide for use in the response measurement method of the present invention may be a GPCR polypeptide expressed by the expression method of the present invention. The GPCR polypeptide is as mentioned above. Specific examples of the GPCR polypeptide preferably include a GPCR polypeptide consisting of an amino acid sequence of any of SEQ ID NOs: 108 to 214 and 275 to 312, or a combination of a GPCR polypeptide consisting of an amino acid sequence of SEQ ID NO: 112 or 113, and a GPCR polypeptide consisting of the amino acid sequence of SEQ ID NO: 114. The GPCR polypeptide can be used in an arbitrary form without losing its responsiveness to a ligand. The GPCR polypeptide can be used in the form of, for example, a transformed cell expressing the GPCR polypeptide or its cultures, a membrane of the transformed cell having the GPCR polypeptide, or an artificial lipid bilayer having the GPCR polypeptide. Preferably, a transformed cell expressing the GPCR polypeptide or its cultures are used as the GPCR polypeptide.

In the response measurement method of the present invention, the measurement of the response of the GPCR polypeptide may be performed by an arbitrary method known in the art as a method for measuring responses of GPCRs. For example, the measurement may be performed by a method for measuring intracellular cAMP levels directly or indirectly. For example, it is known that G protein-coupled receptors, when activated by ligands, are coupled to G protein α subunit classified into the Gs family or G protein α subunit classified into the Gi family in cells to activate or inhibit adenylate cyclase, thereby changing intracellular cAMP levels. Meanwhile, the G protein-coupled receptors, when activated by ligands, can also be coupled to proteins belonging to the Gq family, such as Gαq, Gα16, and Gα15, in cells, thereby increasing calcium ion levels in the cells. Thus, the response of the GPCR polypeptide can be measured by using, as an index, an intracellular calcium ion level, or a behavior of a downstream molecule activated via such a level. Examples of a method for measuring the cAMP level include ELISA and reporter gene assay. Examples of a method for measuring the calcium ion concentration include calcium imaging techniques and TGFα shedding assay. The TGFα shedding assay is also useful to measure signals when GPCRs are coupled to Gα12 and Gα13 in cells. As an example of a method using the behavior of the downstream molecule activated via the cAMP level as an index, a two-electrode voltage clamp technique is also effective which measures potential change inside and outside cell membranes via a cystic fibrosis transmembrane conductance regulator CFTR activated by cAMP signals in *Xenopus* oocytes. Any of the above response measurement methods can be used by utilizing a chimeric protein of a G protein α subunit which is easily coupled to the GPCR of interest and any other G protein α subunit. For example, among various G protein α subunits, taste receptors can be efficiently coupled to those belonging to the Gi family. Based on this fact, by utilizing Gα15 (Gα15/i3, Gα15/gust) having 5 C-terminal amino acids of Gαi3 or Gαgust, signals derived from activated taste receptors can be detected as an increase in intracellular calcium ion concentration rather than a decrease in the original intracellular cAMP level. In addition, a GPCR polypeptide may be purified from cells expressing the GPCR polypeptide according to the present invention and then evaluated for binding to a ligand. An arbitrary method known in the art may be used to evaluate the binding of the purified GPCR polypeptide to a ligand. Examples of such a method include a method of measuring the amount of heat generated from the GPCR polypeptide under ligand addition conditions, and a method for evaluating changes in the thermal stability of the GPCR polypeptide.

As mentioned above, the GPCR polypeptide expressed by the expression method of the present invention can be expressed on a cultured cell membrane more efficiently in comparison with the original GPCR, and a response of the GPCR polypeptide is considered to reflect a response of the original GPCR. Therefore, a ligand for the original GPCR can be searched for using the GPCR polypeptide. Thus, in an alternative aspect, the present invention provides a method for searching for a ligand for a GPCR of interest. The method includes: measuring a response of a GPCR polypeptide expressed by the expression method of the present invention in the presence of a test substance; and selecting a test substance to which the GPCR polypeptide has responded. The ligand search method of the present invention can improve search efficiency of a ligand for a GPCR of interest and enables search for a ligand for a GPCR of interest never before possible to functionally analyze due to insufficient cell membrane expression in cultured cells.

The test substance for use in the ligand search method of the present invention is not particularly limited as long as it is a substance for which it is desired to confirm whether or not to be a ligand for a GPCR of interest. The test substance may be a naturally occurring substance or a substance artificially synthesized by a chemical or biological method or the like, or may be a compound, a composition, or a mixture.

The GPCR polypeptide for use in the ligand search method of the present invention and its form are the same as the GPCR polypeptide for use in the response measurement method of the present invention and its form.

In the ligand search method of the present invention, the test substance is applied to the GPCR polypeptide expressed by the expression method of the present invention. Examples of an approach of applying the test substance to the GPCR polypeptide include, but are not particularly limited to, a method of adding the test substance to a medium for culture of cells expressing the GPCR polypeptide.

In the ligand search method of the present invention, a response of the GPCR polypeptide to the test substance is measured following the addition of the test substance to the GPCR polypeptide. The same approach as that for the response measurement method of the present invention can be used as a method for measuring the response.

Subsequently, the test substance is evaluated on the basis of the measured response of the GPCR polypeptide. A test substance which causes the response of the GPCR polypeptide can be determined as a ligand for the GPCR polypeptide, i.e., a ligand for the original GPCR. Thus, in the ligand search method of the present invention, a test substance to which the GPCR polypeptide has responded is selected as a ligand for the original GPCR.

Preferably, the response of the GPCR polypeptide to the test substance can be evaluated by comparing the response of the GPCR polypeptide supplemented with the test substance (test group) with a response of the GPCR polypeptide in a control group. Examples of the control group can include the GPCR polypeptide non-supplemented with the test substance, the GPCR polypeptide supplemented with a control substance, the GPCR polypeptide supplemented with a lower concentration of the test substance, and the GPCR polypeptide before addition of the test substance. When the response in the test group is higher than that in the control group, the GPCR polypeptide is evaluated as having responded to the test substance and the test substance is selected as a ligand for the original GPCR.

Thus, in one embodiment of the ligand search method of the present invention, a response of the GPCR polypeptide is measured in the presence and in the absence of a test substance, and subsequently, whether or not the response in the presence of the test substance is higher than that in the absence of the test substance is determined. When the response in the presence of the test substance is higher, the test substance is selected as a ligand. In a preferred embodiment, provided that the response intensity of the GPCR polypeptide in the presence of the test substance is preferably 120% or more, more preferably 150% or more, further more preferably 200% or more, in comparison with that in the absence of the test substance, the test substance is selected as a ligand for the original GPCR. In another preferred embodiment, provided that the response intensity of the GPCR polypeptide in the presence of the test substance is statistically significantly increased in comparison with that in the absence of the test substance, the test substance is selected as a ligand for the original GPCR.

As mentioned above, the GPCR polypeptide expressed by the expression method of the present invention can be expressed on a cultured cell membrane more efficiently in comparison with the original GPCR, and a response of the GPCR polypeptide is considered to reflect a response of the original GPCR. Therefore, a substance which controls the recognition of a ligand for the original GPCR can be evaluated and/or selected using the GPCR polypeptide. Thus, in an alternative aspect, the present invention provides a method for evaluating and/or selecting a control agent for recognition of a ligand for a GPCR receptor of interest. The method includes adding a test substance and a target ligand to a GPCR polypeptide expressed by the expression method of the present invention, and measuring a response of the GPCR polypeptide to the ligand. As one example, since vomeronasal receptors are considered to function as pheromone receptors, when a vomeronasal receptor polypeptide is used as the GPCR polypeptide, the ligand is preferably a pheromone substance. As another example, when a taste receptor polypeptide is used as the GPCR polypeptide, the ligand is preferably a taste substance (a bitterness substance, an umami substance, or a sweetness substance). The ligand for a GPCR of interest is preferably a ligand selected by the ligand search method of the present invention. According to the control agent evaluation and/or selection method of the present invention, a substance which can selectively suppress or enhance recognition of the target ligand can be efficiently evaluated or selected.

The control agent evaluation and/or selection of the present invention can be a method which is performed *in vitro* or *ex vivo.*

The test substance for use in the control agent evaluation and/or selection method of the present invention is not particularly limited as long as the substance is desired to be used as a control agent for recognition of the target ligand. The test substance may be a naturally occurring substance or a substance artificially synthesized by a chemical or biological method or the like, or may be a compound, a composition, or a mixture.

The GPCR polypeptide for use in the control agent evaluation and/or selection method of the present invention and its form are the same as the GPCR polypeptide for use in the response measurement method of the present invention and its form.

In the control agent evaluation and/or selection method of the present invention, the test substance and the target ligand are applied to the GPCR polypeptide expressed by the expression method of the present invention. Examples of an approach of applying the test substance and the target ligand to the GPCR polypeptide include, but are not particularly limited to, a method of adding the test substance and the target ligand to a medium for culture of cells expressing the GPCR polypeptide.

In the control agent evaluation and/or selection method of the present invention, a response of the GPCR polypeptide to the target ligand is measured following the addition of the test substance and the ligand to the GPCR polypeptide. The same approach as that for the response measurement method of the present invention can be used as a method for measuring the response.

Subsequently, a test substance which suppresses the response of the GPCR polypeptide to the ligand is detected on the basis of the measured response. The detected test substance is selected as a suppressor of recognition of the ligand. Alternatively, a test substance which enhances the response of the GPCR polypeptide to the ligand is detected on the basis of the measured response. The detected test substance is selected as an enhancer of recognition of the ligand.

The test substance which suppresses the response of the GPCR polypeptide to the ligand is selected as a suppressor of recognition of the ligand. Alternatively, the test substance which enhances the response of the GPCR polypeptide to the ligand is selected as an enhancer of recognition of the ligand. The action of the test substance on the response of the GPCR polypeptide to the ligand can be evaluated, for example, by comparing the response of the GPCR polypeptide supplemented with the test substance (test group) to the ligand with a response to the ligand in a control group. Examples of the control group can include the GPCR polypeptide non-supplemented with the test substance, the GPCR polypeptide supplemented with a control substance, the GPCR polypeptide supplemented with a lower concentration of the test substance, the GPCR polypeptide before addition of the test substance, and cells which do not express the GPCR polypeptide. Preferably, the suppressor evaluation and/or selection method of the present invention includes measuring activity of the GPCR polypeptide against the ligand in the presence and in the absence of the test substance.

For example, when the response in the test group is more suppressed than the response in the control group, the test substance can be identified as a substance which suppresses the response of the GPCR polypeptide to the target ligand, **i.e.,** a substance which suppresses the response of the original GPCR to the ligand. For example, provided that the response of the GPCR polypeptide in the test group is suppressed to preferably 60% or less, more preferably 50% or less, further more preferably 25% or less, in comparison with the control group, the test substance can be identified as a substance which suppresses the response of the GPCR polypeptide to the ligand, **i.e.,** a substance which suppresses the response of the original GPCR to the ligand. Alternatively, provided that the response of the GPCR polypeptide in the test group is statistically significantly suppressed in comparison with the control group, the test substance can be identified as a substance which suppresses the response of the GPCR polypeptide to the ligand, **i.e.,** a substance which suppresses the response of the original GPCR to the ligand.

Alternatively, for example, when the response in the test group is more enhanced than the response in the control group, the test substance can be identified as a substance which enhances the response of the GPCR polypeptide to the target ligand, **i.e.,** a substance which enhances the response of the original GPCR to the ligand. For example, provided that the response of the GPCR polypeptide in the test group is enhanced to preferably 120% or more, more preferably 150% or more, further more preferably 200% or more, in comparison with the control group, the test substance can be identified as a substance which enhances the response of the GPCR polypeptide to the ligand, i.e., a substance which enhances the response of the original GPCR to the ligand. Alternatively, provided that the response of the GPCR polypeptide in the test group is statistically significantly enhanced in comparison with the control group, the test substance can be identified as a substance which enhances the response of the GPCR polypeptide to the ligand, i.e., a substance which enhances the response of the original GPCR to the ligand.

In an embodiment of the control agent evaluation and/or selection method of the present invention, the GPCR polypeptide is a vomeronasal receptor polypeptide expressed by the expression method of the present invention, the ligand for a GPCR of interest is a pheromone substance, and a pheromone suppressor or a pheromone enhancer is evaluated and/or selected as a control agent for recognition of the ligand. In another embodiment of the control agent evaluation and/or selection method of the present invention, the GPCR polypeptide is an umami receptor polypeptide (a combination of consensus TAS1R1 and consensus TAS1R3) expressed by the expression method of the present invention, the ligand for a GPCR of interest is an umami substance, and an umami suppressor or an umami enhancer is evaluated and/or selected as a control agent for recognition of the ligand. In another embodiment of the control agent evaluation and/or selection method of the present invention, the GPCR polypeptide is a sweetness receptor polypeptide (a combination of consensus TAS1R2 and consensus TAS1R3) expressed by the expression method of the present invention, the ligand for a GPCR of interest is a sweetness substance, and a sweetness suppressor or a sweetness enhancer is evaluated and/or selected as a control agent for recognition of the ligand. In another embodiment of the control agent evaluation and/or selection method of the present invention, the GPCR polypeptide is a bitterness receptor polypeptide expressed by the expression method of the present invention, the ligand for a GPCR of interest is a bitterness substance, and a bitterness suppressor or a bitterness enhancer is evaluated and/or selected as a control agent for recognition of the ligand.

The test substance identified by the procedures described above is a substance which can control the response of the GPCR to the target ligand, thereby controlling the recognition of the ligand by an individual. Thus, the test substance identified by the procedures described above can be selected as a control agent for recognition of the ligand. The substance selected as a control agent for recognition of the target ligand by the control agent evaluation and/or selection method of the present invention can control recognition of the ligand by controlling the response of the GPCR to the ligand.

Thus, in one embodiment, the substance selected by the ligand recognition control agent evaluation and/or selection method of the present invention can be an active ingredient for a control agent for recognition of the ligand. Alternatively, the substance selected by the ligand recognition control agent evaluation and/or selection method of the present invention can be contained as an active ingredient for controlling recognition of the ligand in a compound or a composition for controlling recognition of the ligand. Alternatively, the substance selected by the ligand recognition control agent evaluation and/or selection method of the present invention can be used for producing a control agent for recognition of the ligand or for producing a compound or a composition for controlling recognition of the ligand. The substance can suppress or enhance recognition of the target ligand.

As mentioned above, the GPCR polypeptide expressed by the expression method of the present invention can be expressed on a cultured cell membrane more efficiently in comparison with the original GPCR, and a response of the GPCR polypeptide is considered to reflect a response of the original GPCR. Therefore, the taste of a test substance can be evaluated by using a taste receptor polypeptide as the GPCR polypeptide. Thus, in an alternative aspect, the present invention provides a method for evaluating taste. The method includes adding a test substance to a GPCR polypeptide expressed by the expression method of the present invention, and measuring a response of the GPCR polypeptide to the test substance, wherein the GPCR polypeptide is a taste receptor polypeptide. According to the taste evaluation method of the present invention, the taste of the test substance can be efficiently evaluated.

The test substance for use in the taste evaluation method of the present invention is not particularly limited as long as it is a substance for which it is desired to confirm the presence or absence of taste (for example, umami, sweetness, or bitterness) or the degree of taste. The test substance may be a naturally occurring substance or a substance artificially synthesized by a chemical or biological method or the like, or may be a compound, a composition, or a mixture.

The GPCR polypeptide for use in the taste evaluation method of the present invention and its form are the same as the GPCR polypeptide for use in the response measurement method of the present invention and its form as long as the GPCR polypeptide is a taste receptor polypeptide.

In the taste evaluation method of the present invention, the test substance is applied to the GPCR polypeptide expressed by the expression method of the present invention. Examples of an approach of applying the test substance to the GPCR polypeptide include, but are not particularly limited to, a method of adding the test substance to a medium for culture of cells expressing the GPCR polypeptide.

In the taste evaluation method of the present invention, a response of the GPCR polypeptide to the test substance is measured following the addition of the test substance to the GPCR polypeptide. The same approach as that for the response measurement method of the present invention can be used as a method for measuring the response.

Subsequently, the test substance is evaluated on the basis of the measured response of the GPCR polypeptide. A test substance which causes the response of the GPCR polypeptide can be evaluated as a substance which gives taste (for example, umami, sweetness, or bitterness). As the response intensity caused by the test substance is higher, the taste of the test substance is evaluated as greater (for example, stronger umami, stronger sweetness, or stronger bitterness).

Preferably, the response of the GPCR polypeptide to the test substance can be evaluated by comparing the response of the GPCR polypeptide supplemented with the test substance (test group) with a response of the GPCR polypeptide in a control group. Examples of the control group can include the GPCR polypeptide non-supplemented with the test substance, the GPCR polypeptide supplemented with a control substance, the GPCR polypeptide supplemented with a lower concentration of the test substance, and the GPCR polypeptide before addition of the test substance. When the response in the test group is higher than that in the control group, the test substance is evaluated as a substance which gives taste.

Thus, in one embodiment of the taste evaluation method of the present invention, a response of the GPCR polypeptide is measured in the presence and in the absence of a test substance, and subsequently, whether or not the response in the presence of the test substance is higher than that in the absence of the test substance is determined. When the response in the presence of the test substance is higher, the test substance is evaluated as a substance which gives taste. In a preferred embodiment, provided that the response intensity of the GPCR polypeptide in the presence of the test substance is preferably 120% or more, more preferably 150% or more, further more preferably 200% or more, in comparison with that in the absence of the test substance, the test substance is evaluated as a substance which gives taste. In another preferred embodiment, provided that the response intensity of the GPCR polypeptide in the presence of the test substance is statistically significantly increased in comparison with that in the absence of the test substance, the test substance is evaluated as a substance which gives taste.

In one embodiment of the taste evaluation method of the present invention, the GPCR polypeptide is an umami receptor polypeptide (a combination of consensus TAS1R1 and consensus TAS1R3) expressed by the expression method of the present invention, and the taste to be evaluated is umami. In another embodiment of the taste evaluation method of the present invention, the GPCR polypeptide is a sweetness receptor polypeptide (a combination of consensus TAS1R2 and consensus TAS1R3) expressed by the expression method of the present invention, and the taste to be evaluated is sweetness. In another embodiment of the taste evaluation method of the present invention, the GPCR polypeptide is a bitterness receptor polypeptide expressed by the expression method of the present invention, and the taste to be evaluated is bitterness.

As mentioned above, the GPCR polypeptide expressed by the expression method of the present invention can be expressed on a cultured cell membrane more efficiently in comparison with the original GPCR, and a response of the GPCR polypeptide is considered to reflect a response of the original GPCR. Therefore, a substance which suppresses the odor recognition of a ligand (odorant, specifically amine) for the original GPCR can be evaluated and/or selected by using a trace amine-associated receptor polypeptide as the GPCR polypeptide. A substance which suppresses the response of the GPCR polypeptide can cause change in the ligand response of the GPCR polypeptide, **i.e.,** cause change in the ligand response of the original GPCR, and consequently selectively suppress odor of the ligand on the basis of receptor antagonism. On the other hand, a substance which enhances the response of the GPCR polypeptide can cause change in the ligand response of the GPCR polypeptide, i.e., cause change in the ligand response of the original GPCR, and consequently selectively suppress odor of the ligand on the basis of odor cross-adaptation ascribable to receptor agonism.

Thus, in an alternative aspect, the present invention provides a method for evaluating and/or selecting a suppressor of odor of a ligand for a GPCR of interest. The method includes measuring a response of the GPCR polypeptide after addition of a test substance, wherein the GPCR polypeptide is a trace amine-associated receptor polypeptide. A test substance which suppresses or enhances the response of the GPCR polypeptide is detected on the basis of the measured response. The detected test substance is selected as a suppressor of odor of the target ligand. Specifically, a test substance which suppresses the response of the GPCR polypeptide is selected as a suppressor of odor of the ligand based on receptor antagonism, and a test substance which enhances the response of the GPCR polypeptide is selected as a suppressor of odor of the ligand based on odor cross-adaptation ascribable to receptor agonism. The ligand for a GPCR of interest is preferably a ligand selected by the ligand search method of the present invention. The odor suppressor evaluation and/or selection method of the present invention can efficiently evaluate or select a substance which can selectively suppress odor of the target ligand, and can evaluate or select a substance which can selectively suppress odor of the target ligand even if the target ligand is a ligand for a GPCR never before possible to functionally analyze due to insufficient cell membrane expression in cultured cells.

The odor suppressor evaluation and/or selection of the present invention can be a method which is performed *in vitro* or *ex vivo.*

The test substance for use in the odor suppressor evaluation and/or selection method of the present invention is not particularly limited as long as the substance is desired to be used as a suppressor of odor of the target ligand. The test substance may be a naturally occurring substance or a substance artificially synthesized by a chemical or biological method or the like, or may be a compound, a composition, or a mixture.

The GPCR polypeptide for use in the odor suppressor evaluation and/or selection method of the present invention and its form are the same as the GPCR polypeptide for use in the response measurement method of the present invention and its form as long as the GPCR polypeptide is a trace amine-associated receptor polypeptide.

In the odor suppressor evaluation and/or selection method of the present invention, the test substance is applied to the GPCR polypeptide expressed by the expression method of the present invention. Examples of an approach of applying the test substance to the GPCR polypeptide include, but are not particularly limited to, a method of adding the test substance to a medium for culture of cells expressing the GPCR polypeptide.

In the odor suppressor evaluation and/or selection method of the present invention, a response of the GPCR polypeptide to the test substance is measured following the addition of the test substance to the GPCR polypeptide. The same approach as that for the response measurement method of the present invention can be used as a method for measuring the response.

In a first embodiment, the odor suppressor evaluation and/or selection method of the present invention comprises: adding a test substance and a target ligand to a GPCR polypeptide expressed by the expression method of the present invention; and measuring a response of the GPCR polypeptide to the ligand. The same approach as that for the method for applying the test substance can be used as a method for applying the ligand to the GPCR polypeptide. Subsequently, a test substance which suppresses the response of the GPCR polypeptide to the ligand is detected on the basis of the measured response. The detected test substance is selected as a suppressor of odor of the ligand.

In the first embodiment, the test substance which suppresses the response of the GPCR polypeptide to the ligand is selected as a suppressor of odor of the ligand based on receptor antagonism. The action of the test substance on the response of the GPCR polypeptide to the ligand can be evaluated, for example, by comparing the response of the GPCR polypeptide supplemented with the test substance (test group) to the ligand with a response to the ligand in a control group. Examples of the control group can include the GPCR polypeptide non-supplemented with the test substance, the GPCR polypeptide supplemented with a control substance, the GPCR polypeptide supplemented with a lower concentration of the test substance, the GPCR polypeptide before addition of the test substance, and cells which do not express the GPCR polypeptide. Preferably, the odor suppressor evaluation and/or selection method of the present invention according to the first embodiment includes measuring activity of the GPCR polypeptide against the ligand in the presence and in the absence of the test substance.

For example, when the response in the test group is more suppressed than the response in the control group, the test substance can be identified as a substance which suppresses the response of the GPCR polypeptide to the target ligand, i.e., a substance which suppresses the response of the original GPCR to the ligand. For example, provided that the response of the GPCR polypeptide in the test group is suppressed to preferably 60% or less, more preferably 50% or less, further more preferably 25% or less, in comparison with the control group, the test substance can be identified as a substance which suppresses the response of the GPCR polypeptide to the ligand, i.e., a substance which suppresses the response of the original GPCR to the ligand. Alternatively, provided that the response of the GPCR polypeptide in the test group is statistically significantly suppressed in comparison with the control group, the test substance can be identified as a substance which suppresses the response of the GPCR polypeptide to the ligand, i.e., a substance which suppresses the response of the original GPCR to the ligand.

In a second embodiment, the odor suppressor evaluation and/or selection method of the present invention includes: adding a test substance to a GPCR polypeptide expressed by the expression method of the present invention; and measuring a response of the GPCR polypeptide to the test substance. Subsequently, a test substance which enhances the response of the GPCR polypeptide to the target ligand is detected on the basis of the measured response. The detected test substance is selected as a suppressor of odor of the ligand.

The test substance which enhances the response of the GPCR polypeptide can first enhance the response of the GPCR, thereby weakening the response of the GPCR when later exposed to the target ligand. As a result, the odor recognition of the ligand by an individual can be suppressed on the basis of odor cross-adaptation. Thus, in the second embodiment, a suppressor of odor of the ligand based on odor cross-adaptation ascribable to receptor agonism is selected.

The action of the test substance on the GPCR polypeptide can be evaluated, for example, by comparing the response of the GPCR polypeptide supplemented with the test substance (test group) with a response in a control group. Examples of the control group include those mentioned above. Preferably, the odor suppressor evaluation and/or selection method of the present invention according to the second embodiment includes measuring activity of the GPCR polypeptide in the presence and in the absence of the test substance. Also preferably, the odor suppressor evaluation and/or selection method of the present invention according to the second embodiment includes measuring responses of a cell expressing the GPCR polypeptide and a non-expressing cell to the ligand in the presence of the test substance.

For example, when the response in the test group is enhanced in comparison with the control group, the test substance can be identified as a substance which suppresses the response of the GPCR polypeptide to the target ligand, i.e., a substance which suppresses the response of the original GPCR to the ligand. For example, provided that the response of the GPCR polypeptide in the test group is enhanced to preferably 120% or more, more preferably 150% or more, further more preferably 200%, in comparison with the control group, the test substance can be identified as a substance which suppresses the response of the GPCR polypeptide to the ligand, i.e., a substance which suppresses the response of the original GPCR to the ligand. Alternatively, provided that the response of the GPCR polypeptide in the test group is statistically significantly enhanced in comparison with the control group, the test substance can be identified as a substance which suppresses the response of the GPCR polypeptide to the ligand, i.e., a substance which suppresses the response of the original GPCR to the ligand.

The test substance identified by the procedures described above is a substance which can suppress the response of the GPCR to the target ligand, thereby suppressing the odor recognition of the ligand by an individual. Thus, the test substance identified by the procedures described above can be selected as a suppressor of odor of the ligand. The substance selected as a suppressor of odor of the target ligand by the odor suppressor evaluation and/or selection method of the present invention can suppress odor of the ligand by suppressing the response of the GPCR to the ligand.

Thus, in one embodiment, the substance selected by the ligand odor suppressor evaluation and/or selection method of the present invention can be an active ingredient for a suppressor of odor of the ligand. Alternatively, the substance selected by the ligand odor suppressor evaluation and/or selection method of the present invention can be contained as an active ingredient for suppressing odor of the ligand in a compound or a composition for suppressing odor of the ligand. Alternatively, the substance selected by the ligand odor suppressor evaluation and/or selection method of the present invention can be used for producing a suppressor of odor of the ligand or for producing a compound or a composition for suppressing odor of the ligand. The substance can eliminate odor of the target ligand without causing problems, such as discomfort based on strong odor of an aromatic agent and suppression of other odors, which arise in an odor elimination method using a conventional deodorant or aromatic agent.

The present specification further discloses the following compositions, production methods, uses, or methods as exemplary embodiments of the present invention. However, the present invention is not limited by these embodiments.

[1] A method for expressing a GPCR polypeptide, comprising:
expressing, in a cell, a GPCR polypeptide consisting of an amino acid sequence obtained by, in an amino acid sequence of a GPCR of interest (provided that an olfactory receptor is excluded), altering at least one amino acid residue different from that in a consensus amino acid sequence to an amino acid residue at a position corresponding thereto in the consensus amino acid sequence, wherein
the consensus amino acid sequence is an amino acid sequence derived by alignment of the amino acid sequence of the GPCR of interest and amino acid sequences of GPCRs encoded by orthologs of the GPCR of interest in vertebrates.

[2] The method according to [1] which is a method for improving expression of the GPCR polypeptide.
[3] A method for functionalizing a GPCR of interest, comprising:
expressing, in a cell, a GPCR polypeptide consisting of an amino acid sequence obtained by, in an amino acid sequence of a GPCR of interest (provided that an olfactory receptor is excluded), altering at least one amino acid residue different from that in a consensus amino acid sequence to an amino acid residue at a position corresponding thereto in the consensus amino acid sequence, wherein
the consensus amino acid sequence is an amino acid sequence derived by alignment of the amino acid sequence of the GPCR of interest and amino acid sequences of GPCRs encoded by orthologs of the GPCR of interest in vertebrates.

[4] The method according to any one of [1] to [3], wherein the orthologs are orthologs selected from the group consisting of orthologs in mammals, Aves, Reptilia, Amphibia, and fish, preferably orthologs selected from the group consisting of orthologs in mammals, Aves, Reptilia, and Amphibia, more preferably orthologs in mammals.
[5] The method according to any one of [1] to [4], wherein the consensus amino acid sequence is an amino acid sequence containing consensus residues identified in accordance with the following criteria (i) to (iii) from the alignment:
(i) at each amino acid position of the alignment,
   (i-i) when there exists one amino acid residue which is different from the amino acid residue of the GPCR of interest and has a frequency of appearance of 50% or more, the amino acid residue is identified as a consensus residue,
   (i-ii) when there exist two amino acid residues having a frequency of appearance of 50%, the amino acid residue of the GPCR of interest is identified as a consensus residue,
   (i-iii) when there exists an amino acid residue in the GPCR of interest and there exists no amino acid residue having a frequency of appearance of 40% or more, the absence of a consensus residue is identified,
   (i-iv) when there exists no amino acid residue in the GPCR of interest and there exists an amino acid residue having a frequency of appearance of 60% or more, an amino acid residue having the highest frequency of appearance is identified as a consensus residue, and when there exist two or more amino acid residues having the highest frequency of appearance, an amino acid residue having the smallest molecular weight among the amino acid residues is identified as a consensus residue, and
   (i-v) if none of the above (i-i) to (i-iv) is appropriate, the amino acid residue of the GPCR of interest is identified as a consensus residue;
(ii) when the consensus residues are identified in accordance with the criterion (i) and when a consensus residue nearest to the N terminus is a consensus residue at a position corresponding to the N terminus of the GPCR of interest or a C-terminal side thereof and is not a methionine residue, a consensus residue on an N-terminal side of a consensus residue consisting of a methionine residue positioned nearest to the N terminus is changed to the absence of a consensus residue; and
(iii) when the consensus residues are identified in accordance with the criterion (i) and when a consensus residue nearest to the N terminus is a consensus residue at a position corresponding to an N-terminal side of the N terminus of the GPCR of interest and is not a methionine residue, an amino acid residue having the highest frequency of appearance is identified as a consensus residue by tracing back one by one amino acid positions on an N-terminal side of the position of the consensus residue of the alignment until a methionine residue appears, and when there exist two or more amino acid residues having the highest frequency of appearance, an amino acid residue having the smallest molecular weight among the amino acid residues is identified as a consensus residue.

[6] The method according to any one of [1] to [5], wherein the alignment is alignment of the amino acid sequence of the GPCR of interest and amino acid sequences of at least 2, preferably at least 5, more preferably at least 11, further more preferably at least 15, further more preferably at least 30, further more preferably at least 100 GPCRs encoded by orthologs of the GPCR of interest in vertebrates.
[7] The method according to any one of [1] to [6], wherein the GPCR of interest is a human GPCR.
[8] The method according to any one of [1] to [7], wherein the GPCR of interest is a vomeronasal receptor, a taste receptor, a trace amine-associated receptor, a Masrelated G protein-coupled receptor, or a GPR.
[9] The method according to any one of [1] to [8], wherein the GPCR polypeptide preferably consists of an amino acid sequence obtained by, in an amino acid sequence of sequence identification number (2) of a GPCR (1) in the following Tables 2-1 and 2-2, altering at least one amino acid residue different from that in a consensus amino acid sequence of sequence identification number (3) to an amino acid residue at a position corresponding thereto in the consensus amino acid sequence.

**[Table 2-1]**

| No. | (1) GPCR | (2) SEQ ID NO of amino acid sequence | (3) SEQ ID NO of consensus amino acid sequence | No. | (1) GPCR | (2) SEQ ID NO of amino acid sequence | (3) SEQ ID NO of consensus amino acid sequence |
|---|---|---|---|---|---|---|---|
| 1 | VN1R1 | 1 | 108 | 55 | GPR21 | 55 | 162 |
| 2 | VN1R2 | 2 | 109 | 56 | GPR22 | 56 | 163 |
| 3 | VN1R4 | 3 | 110 | 57 | GPR25 | 57 | 164 |
| 4 | VN1R5 | 4 | 111 | 58 | GPR26 | 58 | 165 |
| 5 | TAS1R1 | 5 | 112 | 59 | GPR27 | 59 | 166 |
| 6 | TAS1R2 | 6 | 113 | 60 | GPR31 | 60 | 167 |
| 7 | TAS1R3 | 7 | 114 | 61 | GPR32 | 61 | 168 |
| 8 | TAS2R8 | 8 | 115 | 62 | GPR33 | 62 | 169 |
| 9 | TAS2R16 | 9 | 116 | 63 | GPR34 | 63 | 170 |
| 10 | TAS2R38 | 10 | 117 | 64 | GPR35 | 64 | 171 |
| 11 | TAS2R42 | 11 | 118 | 65 | GPR37 | 65 | 172 |
| 12 | TAS2R45 | 12 | 119 | 66 | GPR37L1 | 66 | 173 |
| 13 | TAS2R46 | 13 | 120 | 67 | GPR39 | 67 | 174 |
| 14 | TAS2R31 | 14 | 121 | 68 | GPR45 | 68 | 175 |
| 15 | TAS2R1 | 15 | 122 | 69 | GPR48 | 69 | 176 |
| 16 | TAS2R3 | 16 | 123 | 70 | GPR49 | 70 | 177 |
| 17 | TAS2R4 | 17 | 124 | 71 | GPR50 | 71 | 178 |
| 18 | TAS2R5 | 18 | 125 | 72 | GPR52 | 72 | 179 |
| 19 | TAS2R7 | 19 | 126 | 73 | GPR61 | 73 | 180 |
| 20 | TAS2R9 | 20 | 127 | 74 | GPR62 | 74 | 181 |
| 21 | TAS2R10 | 21 | 128 | 75 | GPR63 | 75 | 182 |
| 22 | TAS2R13 | 22 | 129 | 76 | GPR65 | 76 | 183 |
| 23 | TAS2R14 | 23 | 130 | 77 | GPR68 | 77 | 184 |
| 24 | TAS2R20 | 24 | 131 | 78 | GPR75 | 78 | 185 |
| 25 | TAS2R30 | 25 | 132 | 79 | GPR78 | 79 | 186 |
| 26 | TAS2R39 | 26 | 133 | 80 | GPR82 | 80 | 187 |
| 27 | TAS2R40 | 27 | 134 | 81 | GPR83 | 81 | 188 |
| 28 | TAS2R43 | 28 | 135 | 82 | GPR84 | 82 | 189 |
| 29 | TAS2R50 | 29 | 136 | 83 | GPR85 | 83 | 190 |
| 30 | TAS2R60 | 30 | 137 | 84 | GPR87 | 84 | 191 |
| 31 | TAAR1 | 31 | 138 | 85 | GPR88 | 85 | 192 |
| 32 | TAAR2 | 32 | 139 | 86 | GPR101 | 86 | 193 |
| 33 | TAAR5 | 33 | 140 | 87 | GPR132 | 87 | 194 |
| 34 | TAAR8 | 34 | 141 | 88 | GPR135 | 88 | 195 |
| 35 | TAAR9 | 35 | 142 | 89 | GPR139 | 89 | 196 |
| 37 | MrgprE | 37 | 144 | 90 | GPR141 | 90 | 197 |
| 38 | MrgprF | 38 | 145 | 91 | GPR142 | 91 | 198 |
| 39 | MAS1 | 39 | 146 | 92 | GPR146 | 92 | 199 |
| 40 | MAS1L | 40 | 147 | 93 | GPR148 | 93 | 200 |
| 41 | MRGPRD | 41 | 148 | 94 | GPR149 | 94 | 201 |
| 42 | MRGPRG | 42 | 149 | 95 | GPR150 | 95 | 202 |
| 43 | MRGPRX1 | 43 | 150 | 96 | GPR151 | 96 | 203 |
| 44 | MRGPRX2 | 44 | 151 | 97 | GPR152 | 97 | 204 |
| 45 | MRGPRX3 | 45 | 152 | 98 | GPR153 | 98 | 205 |
| 46 | MRGPRX4 | 46 | 153 | 99 | GPR160 | 99 | 206 |
| 47 | GPR3 | 47 | 154 | 100 | GPR161 | 100 | 207 |
| 48 | GPR4 | 48 | 155 | 101 | GPR162 | 101 | 208 |
| 49 | GPR6 | 49 | 156 | 102 | GPR171 | 102 | 209 |
| 50 | GPR12 | 50 | 157 | 103 | GPR173 | 103 | 210 |
| 51 | GPR15 | 51 | 158 | 104 | GPR174 | 104 | 211 |
| 52 | GPR17 | 52 | 159 | 105 | GPR176 | 105 | 212 |
| 53 | GPR19 | 53 | 160 | 106 | GPR182 | 106 | 213 |
| 54 | GPR20 | 54 | 161 | 107 | GPR183 | 107 | 214 |

**[Table 2-2]**

| No. | (1) GPCR | (2) SEQ ID NO of amino acid sequence | (3) SEQ ID NO of consensus amino acid sequence | No. | (1) GPCR | (2) SEQ ID NO of amino acid sequence | (3) SEQ ID NO of consensus amino acid sequence |
|---|---|---|---|---|---|---|---|
| 108 | GPR42 | 237 | 275 | 127 | ADGRF5 | 256 | 294 |
| 109 | ADGRA1 | 238 | 276 | 128 | ADGRG1 | 257 | 295 |
| 110 | ADGRA2 | 239 | 277 | 129 | ADGRG2 | 258 | 296 |
| 111 | ADGRA3 | 240 | 278 | 130 | ADGRG4 | 259 | 297 |
| 112 | ADGRB1 | 241 | 279 | 131 | ADGRG5 | 260 | 298 |
| 113 | ADGRB2 | 242 | 280 | 132 | ADGRG6 | 261 | 299 |
| 114 | ADGRB3 | 243 | 281 | 133 | ADGRG7 | 262 | 300 |
| 115 | ADGRC1 | 244 | 282 | 134 | AOGRL1 | 263 | 301 |
| 116 | ADGRC2 | 245 | 283 | 135 | ADGRL2 | 264 | 302 |
| 117 | ADGRC3 | 246 | 284 | 136 | ADGRL3 | 265 | 303 |
| 118 | ADGRD1 | 247 | 285 | 137 | ADGRL4 | 266 | 304 |
| 119 | ADGRD2 | 248 | 286 | 138 | Chrm-4/M4R | 267 | 305 |
| 120 | ADGRE1 | 249 | 287 | 139 | F2RL1 | 268 | 306 |
| 121 | ADGRE2 | 250 | 288 | 140 | GRM5 | 269 | 307 |
| 122 | ADGRE3 | 251 | 289 | 141 | APLNR | 270 | 308 |
| 123 | ADGRE5 | 252 | 290 | 142 | CALCRL | 271 | 309 |
| 124 | ADGRF1 | 253 | 291 | 143 | GLP2R | 272 | 310 |
| 125 | ADGRF3 | 254 | 292 | 144 | MC4R | 273 | 311 |
| 126 | ADGRF4 | 255 | 293 | 145 | CCR6 | 274 | 312 |

[10] The method according to [9], wherein the GPCR polypeptide preferably consists of an amino acid sequence of any of SEQ ID NOs: 108 to 142, 144 to 214, and 275 to 312.
[11] A method for expressing a GPCR polypeptide, comprising:
expressing, in a cell, a GPCR polypeptide consisting of an amino acid sequence obtained by, in an amino acid sequence of a GPCR of interest, altering at least one amino acid residue different from that in a consensus amino acid sequence to an amino acid residue at a position corresponding thereto in the consensus amino acid sequence, wherein
the GPCR polypeptide consists of an amino acid sequence obtained by, in the amino acid sequence of SEQ ID NO: 36 of human TAAR6, altering at least one amino acid residue different from that in the consensus amino acid sequence of SEQ ID NO: 143 to an amino acid residue at a position corresponding thereto in the consensus amino acid sequence, preferably consists of the amino acid sequence of SEQ ID NO: 143.

[12] The method according to [11] which is a method for improving expression of the GPCR polypeptide.
[13] A method for functionalizing a GPCR of interest, comprising:
expressing, in a cell, a GPCR polypeptide consisting of an amino acid sequence obtained by, in an amino acid sequence of a GPCR of interest, altering at least one amino acid residue different from that in a consensus amino acid sequence to an amino acid residue at a position corresponding thereto in the consensus amino acid sequence, wherein
the GPCR polypeptide consists of an amino acid sequence obtained by, in the amino acid sequence of SEQ ID NO: 36 of human TAAR6, altering at least one amino acid residue different from that in the consensus amino acid sequence of SEQ ID NO: 143 to an amino acid residue at a position corresponding thereto in the consensus amino acid sequence, preferably consists of the amino acid sequence of SEQ ID NO: 143.

[14] The method according to any one of [1] to [13], wherein the GPCR polypeptide is expressed on a cell membrane of the cell.
[15] The method according to any one of [1] to [14], preferably further comprising expressing RTP1S in the cell.
[16] The method according to any one of [1] to [15], wherein the cell is a HEK293 cell.
[17] A method for measuring a response of a GPCR of interest, comprising:
measuring a response of the GPCR polypeptide expressed by the method according to any one of [1] to [16].
[18] A method for searching for a ligand for a GPCR of interest, comprising:
measuring a response of the GPCR polypeptide expressed by the method according to any one of [1] to [16] in the presence of a test substance; and
selecting a test substance to which the GPCR polypeptide has responded.

[19] The method according to [18], preferably further comprising measuring a response of the GPCR polypeptide in the absence of the test substance.
[20] The method according to [19], wherein a test substance is preferably selected which increases the response of the GPCR polypeptide in the presence of the test substance to 120% or more of the response in the absence of the test substance.
[21] The method according to [19], wherein a test substance is preferably selected which statistically significantly increases the response of the GPCR polypeptide in the presence of the test substance in comparison with the response in the absence of the test substance.
[22] A method for evaluating and/or selecting a control agent for recognition of a ligand for a GPCR of interest, comprising:
adding a test substance and a ligand for a GPCR of interest to the GPCR polypeptide expressed by the method according to any one of [1] to [16]; and
measuring a response of the GPCR polypeptide to the ligand.

[23] The method according to [22], wherein the ligand is the ligand selected by the method according to any one of [18] to [21].
[24] The method according to [22] or [23], further comprising identifying a test substance which controls, preferably suppresses or enhances, the response of the GPCR polypeptide on the basis of the measured response.
[25] The method according to any one of [22] to [24], further comprising measuring a response of the GPCR polypeptide non-supplemented with the test substance to the ligand.
[26] The method according to [25], further comprising:
identifying the test substance as a substance which suppresses the response of the GPCR polypeptide to the ligand when the response of the GPCR polypeptide supplemented with the test substance to the ligand is more suppressed than the response of the GPCR polypeptide non-supplemented with the test substance to the ligand, or
identifying the test substance as a substance which enhances the response of the GPCR polypeptide to the ligand when the response of the GPCR polypeptide supplemented with the test substance to the ligand is more enhanced than the response of the GPCR polypeptide non-supplemented with the test substance to the ligand.

[27] A method for evaluating taste, comprising:
adding a test substance to the GPCR polypeptide expressed by the method according to any one of [1] to [16]; and
measuring a response of the GPCR polypeptide to the test substance, wherein
the GPCR polypeptide is a taste receptor polypeptide.

[28] The method according to [27], preferably further comprising measuring a response of the GPCR polypeptide non-supplemented with the test substance.
[29] The method according to [28], wherein a test substance which increases the response of the GPCR polypeptide supplemented with the test substance to 120% or more of the response of the GPCR polypeptide non-supplemented with the test substance is preferably evaluated as a substance which gives taste.
[30] The method according to [28], wherein a test substance which statistically significantly increases the response of the GPCR polypeptide supplemented with the test substance in comparison with the response of the GPCR polypeptide non-supplemented with the test substance is preferably evaluated as a substance which gives taste.
[31] The method according to any one of [27] to [30], wherein the taste is preferably umami, sweetness, or bitterness.
[32] A method for evaluating and/or selecting a suppressor of odor of a ligand for a GPCR of interest, comprising:
adding a test substance and a ligand for a GPCR of interest to the GPCR polypeptide expressed by the method according to any one of [1] to [16]; and
measuring a response of the GPCR polypeptide to the ligand, wherein
the GPCR polypeptide is a trace amine-associated receptor polypeptide.

[33] The method according to [32], wherein the ligand is the ligand selected by the method according to any one of [18] to [21].
[34] The method according to [32] or [33], further comprising identifying a test substance which suppresses the response of the GPCR polypeptide on the basis of the measured response.
[35] The method according to any one of [32] to [34], further comprising measuring a response of the GPCR polypeptide non-supplemented with the test substance to the ligand.
[36] The method according to [35], further comprising identifying the test substance as a substance which suppresses the response of the GPCR polypeptide to the ligand when the response of the GPCR polypeptide supplemented with the test substance to the ligand is more suppressed than the response of the GPCR polypeptide non-supplemented with the test substance to the ligand.
[37] A method for evaluating and/or selecting a suppressor of odor of a ligand for a GPCR of interest, comprising:
adding a test substance to the GPCR polypeptide expressed by the method according to any one of [1] to [16]; and
measuring a response of the GPCR polypeptide to the test substance, wherein
the GPCR polypeptide is a trace amine-associated receptor polypeptide.

[38] The method according to [37], wherein the ligand is the ligand selected by the method according to any one of [18] to [21].
[39] The method according to [37] or [38], further comprising identifying a test substance which enhances the response of the GPCR polypeptide on the basis of the measured response.
[40] The method according to any one of [37] to [39], further comprising measuring a response of the GPCR polypeptide non-supplemented with the test substance.
[41] The method according to [40], further comprising identifying the test substance as a substance which suppresses the response of the GPCR polypeptide to the ligand when the response of the GPCR polypeptide supplemented with the test substance is more enhanced than the response of the GPCR polypeptide non-supplemented with the test substance.
[42] The method according to any one of [17] to [41], wherein the response of the GPCR polypeptide is preferably measured through intracellular cAMP level measurement by ELISA or reporter gene assay, calcium ion level measurement by calcium imaging or TGFα shedding assay, or potential change measurement inside and outside cell membranes by a two-electrode voltage clamp technique using *Xenopus* oocytes.
[43] An altered GPCR polypeptide consisting of an amino acid sequence obtained by, in an amino acid sequence of a GPCR of interest (provided that an olfactory receptor is excluded), altering at least one amino acid residue different from that in a consensus amino acid sequence to an amino acid residue at a position corresponding thereto in the consensus amino acid sequence, wherein
the consensus amino acid sequence is an amino acid sequence derived by alignment of the amino acid sequence of the GPCR of interest and amino acid sequences of GPCRs encoded by orthologs of the GPCR of interest in vertebrates.
[44] The altered GPCR polypeptide according to [43], wherein the orthologs are orthologs selected from the group consisting of orthologs in mammals, Aves, Reptilia, Amphibia, and fish, preferably orthologs selected from the group consisting of orthologs in mammals, Aves, Reptilia, and Amphibia, more preferably orthologs in mammals.
[45] The altered GPCR polypeptide according to [43] or [44], wherein the consensus amino acid sequence is an amino acid sequence containing consensus residues identified in accordance with the following criteria (i) to (iii) from the alignment:
(i) at each amino acid position of the alignment,
   (i-i) when there exists one amino acid residue which is different from the amino acid residue of the GPCR of interest and has a frequency of appearance of 50% or more, the amino acid residue is identified as a consensus residue,
   (i-ii) when there exist two amino acid residues having a frequency of appearance of 50%, the amino acid residue of the GPCR of interest is identified as a consensus residue,
   (i-iii) when there exists an amino acid residue in the GPCR of interest and there exists no amino acid residue having a frequency of appearance of 40% or more, the absence of a consensus residue is identified,
   (i-iv) when there exists no amino acid residue in the GPCR of interest and there exists an amino acid residue having a frequency of appearance of 60% or more, an amino acid residue having the highest frequency of appearance is identified as a consensus residue, and when there exist two or more amino acid residues having the highest frequency of appearance, an amino acid residue having the smallest molecular weight among the amino acid residues is identified as a consensus residue, and
   (i-v) if none of the above (i-i) to (i-iv) is appropriate, the amino acid residue of the GPCR of interest is identified as a consensus residue;
(ii) when the consensus residues are identified in accordance with the criterion (i) and when a consensus residue nearest to the N terminus is a consensus residue at a position corresponding to the N terminus of the GPCR of interest or a C-terminal side thereof and is not a methionine residue, a consensus residue on an N-terminal side of a consensus residue consisting of a methionine residue positioned nearest to the N terminus is changed to the absence of a consensus residue; and
(iii) when the consensus residues are identified in accordance with the criterion (i) and when a consensus residue nearest to the N terminus is a consensus residue at a position corresponding to an N-terminal side of the N terminus of the GPCR of interest and is not a methionine residue, an amino acid residue having the highest frequency of appearance is identified as a consensus residue by tracing back one by one amino acid positions on an N-terminal side of the position of the consensus residue of the alignment until a methionine residue appears, and when there exist two or more amino acid residues having the highest frequency of appearance, an amino acid residue having the smallest molecular weight among the amino acid residues is identified as a consensus residue.

[46] The altered GPCR polypeptide according to any one of [43] to [45], wherein the alignment is alignment of the amino acid sequence of the GPCR of interest and amino acid sequences of at least 2, preferably at least 5, more preferably at least 11, further more preferably at least 15, further more preferably at least 30, further more preferably at least 100 GPCRs encoded by orthologs of the GPCR of interest in vertebrates.
[47] The altered GPCR polypeptide according to any one of [43] to [46], wherein the GPCR of interest is a human GPCR.
[48] The altered GPCR polypeptide according to any one of [43] to [47], wherein the GPCR of interest is a vomeronasal receptor, a taste receptor, a trace amine-associated receptor, a Mas-related G protein-coupled receptor, or a GPR.
[49] The altered GPCR polypeptide according to any one of [43] to [48], which preferably consists of an amino acid sequence obtained by, in an amino acid sequence of sequence identification number (2) of a GPCR (1) in the above Tables 2-1 and 2-2, altering at least one amino acid residue different from that in a consensus amino acid sequence of sequence identification number (3) to an amino acid residue at a position corresponding thereto in the consensus amino acid sequence.
[50] The altered GPCR polypeptide according to [49], which preferably consists of an amino acid sequence of any of SEQ ID NOs: 108 to 142, 144 to 214, and 275 to 312.
[51] An altered GPCR polypeptide consisting of an amino acid sequence obtained by, in the amino acid sequence of SEQ ID NO: 36 of human TAAR6, altering at least one amino acid residue different from that in the consensus amino acid sequence of SEQ ID NO: 143 to an amino acid residue at a position corresponding thereto in the consensus amino acid sequence, preferably consists of the amino acid sequence of SEQ ID NO: 143.
[52] A polynucleotide encoding the altered GPCR polypeptide according to any one of [43] to [51].
[53] The polynucleotide according to [52], which consists of a nucleotide sequence of any of SEQ ID NOs: 215 to 234 and 313 to 317.
[54] A vector or a DNA fragment comprising the polynucleotide according to [52] or [53].
[55] A transformed cell comprising the vector or the DNA fragment according to [54].
[56] The transformed cell according to [55], preferably further comprising a vector or a DNA fragment comprising a polynucleotide encoding RTP1S.
[57] The transformed cell according to [55] or [56], wherein the cell is a HEK293 cell.

### Examples

Hereinafter, the present invention will be more specifically described with reference to Examples.

### Example 1 Preparation and analysis of consensus receptor

### 1) Preparation of receptor gene

For consensus receptor design, homologous gene candidates of the gene of a receptor of interest were searched for using NCBI BLAST. An ortholog group was identified as to the obtained gene group.

Specifically, when the receptor of interest was a human receptor (GPCR), the identification of orthologs was carried out by selecting genes having the same name as that of the receptor of interest as orthologs from top homologous genes searched for by BLAST.

For example, in the case of TAAR1, which is a human trace amine-associated receptor, 249 genes involving TAAR1 in their names were identified as orthologs from among top 250 homologous genes searched for by BLAST setting the amino acid sequence of human TAAR1 (NP_612200.1) to a query sequence. The amino acid sequences of a total of 250 genes (Tables 3-1 to 4) consisting of these 249 genes plus human TAAR1 were subjected to alignment analysis and consensus amino acid identification as mentioned below. As for human TAAR2, TAAR5, TAAR6, TAAR8, and TAAR9, an ortholog group was identified in the same manner as above. When the receptor of interest was human TAAR6, TAAR8, or TAAR9, the identified orthologs were orthologs in primates and orthologs in orders of mammals other than primates. When the receptor of interest was TAAR2 or TAAR5, the identified orthologs were orthologs in primates, orthologs in orders of mammals other than primates, and orthologs in Aves. When the receptor of interest was TAAR1, the identified orthologs were orthologs in primates, orthologs in orders of mammals other than primates, orthologs in Aves, orthologs in Reptilia, and orthologs in Amphibia.

For example, in the case of VN1R1, which is a human vomeronasal receptor, when a BLAST search was performed by setting the amino acid sequence of human VN1R1 (NP_065684.1) to a query sequence, 22 genes involving vomeronasal type-1 receptor 1 in their names were identified from among top 250 homologous genes. VN1Rs are a family of receptor genes with high diversity among homologous genes, and among those homologous genes, 7 genes with an amino acid identity of 65% or more to human VN1R1 were identified as orthologs. The amino acid sequences of a total of 8 genes consisting of these 7 genes plus human VN1R1 were subjected to alignment analysis and consensus amino acid identification as mentioned below. As for human VN1R2, VN1R4, and VN1R5, an ortholog group was identified in the same manner as above. When the receptor of interest was VN1R1, VN1R2, VN1R4, or VN1R5, the identified orthologs were orthologs in mammals (primates).

For example, in the case of TAS1R1, which is a human taste receptor (umami receptor), 249 genes involving TAS1R1 in their names were identified as orthologs from among top 250 homologous genes searched for by BLAST setting the amino acid sequence of human TAS1R1 (NP_619642.2) to a query sequence. The amino acid sequences of a total of 250 genes consisting of these 249 genes plus human TAS1R1 were subjected to alignment analysis and consensus amino acid identification as mentioned below. As for human TAS1R2 and TAS1R3, an ortholog group was identified in the same manner as above. When the receptor of interest was TAS1R1, TAS1R2, or TAS1R3, the identified orthologs were orthologs in primates, orthologs in orders of mammals other than primates, orthologs in Aves, orthologs in Reptilia, orthologs in Amphibia, and orthologs in fish.

For example, in the case of TAS2R8, which is a human taste receptor (bitterness receptor), 90 genes involving TAS2R8 in their names were identified as orthologs from among top 250 homologous genes searched for by BLAST setting the amino acid sequence of human TAS2R8 (NP_076407.1) to a query sequence. The amino acid sequences of a total of 91 genes consisting of these 90 genes plus human TAS2R8 were subjected to alignment analysis and consensus amino acid identification as mentioned below. As for human TAS2R16, TAS2R38, TAS2R42, TAS2R45, TAS2R46, TAS2R31, TAS2R1, TAS2R3, TAS2R4, TAS2R5, TAS2R7, TAS2R9, TAS2R10, TAS2R13, TAS2R14, TAS2R20, TAS2R30, TAS2R39, TAS2R40, TAS2R43, TAS2R50, and TAS2R60, an ortholog group was identified in the same manner as above. When the receptor of interest was human TAS2R45, the identified orthologs were orthologs in primates; when the receptor of interest was human TAS2R8, TAS2R16, TAS2R38, TAS2R42, or TAS2R46, the identified orthologs were orthologs in primates and orthologs in orders of mammals other than primates.

For example, in the case of MrgprE, which is a human Mas-related G protein-coupled receptor, 72 genes involving MrgprE in their names were identified as orthologs from among top 250 homologous genes searched for by BLAST setting the amino acid sequence of human MrgprE (NP_001034254.2) to a query sequence. The amino acid sequences of a total of 73 genes consisting of these 72 genes plus human MrgprE were subjected to alignment analysis and consensus amino acid identification as mentioned below. As for human MrgprF, MAS1, MAS1L, MRGPRD, MRGPRG, MRGPRX1, MRGPRX2, MRGPRX3, and MRGPRX4, an ortholog group was identified in the same manner as above. When the receptor of interest was human MrgprE or MrgprF, the identified orthologs were orthologs in primates and orthologs in orders of mammals other than primates.

For example, in the case of GPR3, which is a human GPR, 234 genes involving GPR3 in their names were identified as orthologs from among top 250 homologous genes searched for by BLAST setting the amino acid sequence of human GPR3 (NP_005272.1) to a query sequence. The amino acid sequences of a total of 235 genes consisting of these 234 genes plus human GPR3 were subjected to alignment analysis and consensus amino acid identification as mentioned below. As for the human GPRs other than GPR3, GPR17, GPR31, GPR50, GPR65, GPR68, and GPR42 shown in Table 4, an ortholog group was identified in the same manner as above. In the case of human GPR17, genes involving "uracil nucleotide/cysteinyl leukotriene receptor", which is known as an ortholog, in their names were identified as orthologs. Similarly, in the case of human GPR31, genes involving "12-(S)-hydroxy-5,8,10,14-eicosatetraenoic acid receptor" in their names; in the case of human GPR50, genes involving "melatonin-related receptor" in their names; in the case of human GPR65, genes involving "psychosine receptor" in their names; and in the case of human GPR68, genes involving "ovarian cancer G-protein coupled receptor 1" in their names were each identified as orthologs. Further, in the case of human GPR42, genes involving "GPR42" in their names and genes involving "free fatty acid receptor 3" in their names (GPCR genes having high homology to GPR42 gene) were identified as orthologs.

Among human GPCRs other than those mentioned above, in the case of ADGRB1, 234 genes involving ADGRB1 in their names were identified as orthologs from among top 250 homologous genes searched for by BLAST setting the amino acid sequence of human ADGRB1 (NP_001693.2) to a query sequence. The amino acid sequences of a total of 235 genes consisting of these 234 genes plus human ADGRB1 were subjected to alignment analysis and consensus amino acid identification as mentioned below. As for human ADGRB2, ADGRB3, and ADGRD1, an ortholog group was identified in the same manner as above. Further, in the case of human ADGRA1, genes involving "ADGRA1" in their names and genes involving "GPR123" in their names (GPCR genes having high homology to ADGRA1 gene) were identified as orthologs. Similarly, in the case of human ADGRA2, genes involving "ADGRA2" in their names and genes involving "GPR124" in their names; and in the case of human ADGRA3, genes involving "ADGRA3" in their names and genes involving "GPR125" in their names were each identified as orthologs. In addition, in the case of human ADGRC1, genes involving "EGF LAG seven-pass G-type receptor 1" in their names (GPCR genes having high homology to ADGRC1 gene) were identified as orthologs. Similarly, in the case of human ADGRC2, genes involving "cadherin EGF LAG seven-pass G-type receptor 2" in their names; in the case of human ADGRC3, genes involving "cadherin EGF LAG seven-pass G-type receptor 3" in their names; in the case of human ADGRD2, genes involving "adhesion G protein-coupled receptor D2" or "G-protein coupled receptor 144" in their names; in the case of human ADGRE1, genes involving "adhesion G protein-coupled receptor E1" or "adhesion G protein-coupled receptor E1 isoform 2 precursor" in their names; in the case of human ADGRE2, genes involving "adhesion G protein-coupled receptor E2" in their names; in the case of human ADGRE3, genes involving "adhesion G protein-coupled receptor E3" in their names; in the case of human ADGRE5, genes involving "adhesion G protein-coupled receptor E5" or "CD97 antigen" in their names; in the case of human ADGRF1, genes involving "adhesion G-protein coupled receptor F1" or "adhesion G-protein coupled receptor F1 isoform 1 precursor" in their names; in the case of human ADGRF3, genes involving "adhesion G-protein coupled receptor F3" or "G-protein coupled receptor 113" in their names; in the case of human ADGRF4, genes involving "adhesion G-protein coupled receptor F4" or "G-protein coupled receptor 115" in their names; in the case of human ADGRF5, genes involving "adhesion G-protein coupled receptor F5" or "G-protein coupled receptor 116" in their names; in the case of human ADGRG1, genes involving "adhesion G-protein coupled receptor G1" or "adhesion G-protein coupled receptor G1 isoform b precursor" in their names; in the case of human ADGRG2, genes involving "adhesion G-protein coupled receptor G2" or "G-protein coupled receptor 64" in their names; in the case of human ADGRG4, genes involving "adhesion G-protein coupled receptor G4" or "G-protein coupled receptor 112" in their names; in the case of human ADGRG5, genes involving "adhesion G-protein coupled receptor G5" or "G-protein coupled receptor 114" in their names; in the case of human ADGRG6, genes involving "adhesion G-protein coupled receptor G6" or "G-protein coupled receptor 126" in their names; in the case of human ADGRG7, genes involving "adhesion G-protein coupled receptor G7" or "adhesion G-protein coupled receptor G7 isoform 2 precursor" in their names; in the case of human ADGRL1, genes involving "adhesion G protein-coupled receptor L1" or "adhesion G protein-coupled receptor L1 isoform 1 precursor" in their names; in the case of human ADGRL2, genes involving "adhesion G-protein coupled receptor L2" or "latrophilin and seven transmembrane domain-containing protein" in their names; in the case of human ADGRL3, genes involving "adhesion G-protein coupled receptor L3" or "latrophilin-3" in their names; in the case of human ADGRL4, genes involving "adhesion G-protein coupled receptor L4" or "latrophilin-2 isoform" in their names; in the case of human Chrm-4/M4R, genes involving "M4R" in their names; in the case of human F2RL1, genes involving "proteinase-activated receptor 2" in their names; in the case of human GRM5, genes involving "metabotropic glutamate receptor 5" in their names; in the case of human APLNR, genes involving "apelin receptor" in their names; in the case of human CALCRL, genes involving "calcitonin gene-related peptide type 1" in their names; in the case of human GLP2R, genes involving "glucagon-like peptide 2 receptor" in their names; in the case of human MC4R, genes involving "MC4R", "Melanocortin receptor 4", "Melanocortin-4 receptor", or "Melanocortin 4 receptor" in their names; and in the case of human CCR6, genes involving "C-C chemokine receptor type 6" in their names were each identified as orthologs.

As for each human GPCR, Table 4 shows the human GPCR genes and the total number of the identified orthologs as the number of reference genes.

**[Table 3-1]**

| | Reference gene |
|---|---|
| 1 | trace amine-associated receptor 1 [Homo sapiens] |
| 2 | trace amine-associated receptor 1 [Pan paniscus] |
| 3 | trace amine-associated receptor 1 [Pan troglodytes] |
| 4 | trace amine-associated receptor 1 [Gorilla gorilla gorilla] |
| 5 | trace amine-associated receptor 1 [Hylobates moloch] |
| 6 | trace amine-associated receptor 1 [Macaca mulatta] |
| 7 | PREDICTED: trace amine-associated receptor 1 [Cercocebus atys] |
| 8 | trace amine-associated receptor 1 [Macaca nemestrina] |
| 9 | trace amine-associated receptor 1 [Pongo abelii] |
| 10 | trace amine-associated receptor 1 [Chlorocebus sabaeus] |
| 11 | PREDICTED: trace amine-associated receptor 1 [Mandrillus leucophaeus] |
| 12 | trace amine-associated receptor 1 [Theropithecus gelada] |
| 13 | PREDICTED: trace amine-associated receptor 1 [Colobus angolensis palliatus] |
| 14 | trace amine-associated receptor 1 [Papio anubis] |
| 15 | trace amine-associated receptor 1 [Nomascus leucogenys] |
| 16 | trace amine-associated receptor 1 [Trachypithecus francoisi] |
| 17 | trace amine-associated receptor 1 [Aotus nancymaae] |
| 18 | trace amine-associated receptor 1 [Rhinopithecus roxellana] |
| 19 | LOW QUALITY PROTEIN: trace amine-associated receptor 1-like [Piliocolobus tephrosceles] |
| 20 | PREDICTED: trace amine-associated receptor 1 [Rhinopithecus bieti] |
| 21 | trace amine-associated receptor 1 [Sapajus apella] |
| 22 | trace amine-associated receptor 1 [Cebus imitator] |
| 23 | trace amine-associated receptor 1 [Callithrix jacchus] |
| 24 | trace amine-associated receptor 1 [Saimiri boliviensis boliviensis] |
| 25 | trace amine-associated receptor 1 [Carlito syrichta] |
| 26 | trace amine-associated receptor 1 [Balaenoptera musculus] |
| 27 | trace amine-associated receptor 1 [Balaenoptera acutorostrata scammoni] |
| 28 | trace amine-associated receptor 1 [Loxodonta africana] |
| 29 | trace amine-associated receptor 1 [Physeter catodon] |
| 30 | trace amine-associated receptor 1 [Orcinus orca] |
| 31 | PREDICTED: trace amine-associated receptor 1 [Lipotes vexillifer] |
| 32 | trace amine-associated receptor 1 isoform X2 [Phocoena sinus] |
| 33 | trace amine-associated receptor 1 [Globicephala melas] |
| 34 | trace amine-associated receptor 1 [Tursiops truncatus] |
| 35 | trace amine-associated receptor 1 [Monodon monoceros] |
| 36 | PREDICTED: trace amine-associated receptor 1 [Hipposideros amiger] |
| 37 | trace amine-associated receptor 1 [Rhinolophus ferrumequinum] |
| 38 | trace amine-associated receptor 1 isoform X2 [Neophocaena asiaeorientalis asiaeorientalis] |
| 39 | trace amine-associated receptor 1 [Lagenorhynchus obliquidens] |
| 40 | trace amine-associated receptor 1 isoform X1 [Delphinapterus leucas] |
| 41 | trace amine-associated receptor 1 [Myotis myotis] |
| 42 | trace amine-associated receptor 1 [Microcebus murinus] |
| 43 | trace amine-associated receptor 1 [Molossus molossus] |
| 44 | PREDICTED: trace amine-associated receptor 1 [Propithecus coquereli] |
| 45 | trace amine-associated receptor 1 [Pteropus vampyrus] |
| 46 | trace amine-associated receptor 1 [Pteropus alecto] |
| 47 | trace amine-associated receptor 1 [Otolemur garnettii] |
| 48 | LOW QUALITY PROTEIN: trace amine-associated receptor 1 [Sus scrofa] |
| 49 | trace amine-associated receptor 1 [Desmodus rotundus] |
| 50 | trace amine-associated receptor 1 [Phyllostomus discolor] |
| 51 | trace amine-associated receptor 1 [Eptesicus fuscus] |
| 52 | trace amine-associated receptor 1 [Lontra canadensis] |
| 53 | trace amine-associated receptor 1 [Tupaia chinensis] |
| 54 | trace amine-associated receptor 1 [Ursus arctos horribilis] |
| 55 | trace amine-associated receptor 1 [Dasypus novemcinctus] |
| 56 | trace amine-associated receptor 1 [Ailuropoda melanoleuca] |
| 57 | trace amine-associated receptor 1 [Enhydra lutris kenyoni] |
| 58 | trace amine-associated receptor 1 [Trichechus manatus latirostris] |
| 59 | trace amine-associated receptor 1 [Myotis lucifugus] |
| 60 | PREDICTED: trace amine-associated receptor 1 [Myotis brandtii] |
| 61 | PREDICTED: trace amine-associated receptor 1 [Miniopterus natalensis] |
| 62 | trace amine-associated receptor 1 [Mustela erminea] |
| 63 | trace amine-associated receptor 1 [Vicugna pacos] |
| 64 | trace amine-associated receptor 1 [Zalophus califomianus] |
| 65 | PREDICTED: trace amine-associated receptor 1 [Mustela putorius furo] |

**[Table 3-2]**

| | Reference gene |
|---|---|
| 66 | trace amine-associated receptor 1 [Artibeus jamaicensis] |
| 67 | trace amine-associated receptor 1 [Manis pentadactyla] |
| 68 | PREDICTED: trace amine-associated receptor 1 [Equus przewalskii] |
| 69 | PREDICTED: trace amine-associated receptor 1 [Odobenus rosmarus divergens] |
| 70 | trace amine-associated receptor 1 [Mirounga leonina] |
| 71 | trace amine-associated receptor 1 [Pipistrellus kuhlii] |
| 72 | trace amine-associated receptor 1 [Rousettus aegyptiacus] |
| 73 | trace amine-associated receptor 1 [Leptonychotes weddellii] |
| 74 | trace amine-associated receptor 1 [Manis javanica] |
| 75 | trace amine-associated receptor 1 [Choloepus didactylus] |
| 76 | trace amine-associated receptor 1 [Callorhinus ursinus] |
| 77 | trace amine-associated receptor 1 [Neomonachus schauinslandi] |
| 78 | PREDICTED: trace amine-associated receptor 1 [Chrysochloris asiatica] |
| 79 | trace amine-associated receptor 1 [Echinops telfairi] |
| 80 | trace amine-associated receptor 1 [Phoca vitulina] |
| 81 | trace amine-associated receptor 1 [Myotis lucifugus] |
| 82 | trace amine-associated receptor 1 [Talpa occidentalis] |
| 83 | LOW QUALITY PROTEIN: trace amine-associated receptor 1 [Pteropus giganteus] |
| 84 | trace amine-associated receptor 1 [Halichoerus prypus] |
| 85 | trace amine-associated receptor 1 [Camelus dromedarius] |
| 86 | PREDICTED: trace amine-associated receptor 1 [Ceratotherium simum simum] |
| 87 | trace amine-associated receptor 1 [Oryx dammah] |
| 88 | PREDICTED: LOW QUALITY PROTEIN: trace amine-associated receptor 1-like [Galeopterus variegatus] |
| 89 | trace amine-associated receptor 1 [Suricata suricatta] |
| 90 | trace amine-associated receptor 1 [Ovis aries] |
| 91 | trace amine-associated receptor 1 [Hyaena hyaena] |
| 92 | trace amine-associated receptor 1 [Felis catus] |
| 93 | trace amine-associated receptor 1 [Camelus ferus] |
| 94 | trace amine-associated receptor 1 [Acinonyx jubatus] |
| 95 | trace amine-associated receptor 1 [Puma yagouaroundi] |
| 96 | trace amine-associated receptor 1 [Puma concolor] |
| 97 | PREDICTED: trace amine-associated receptor 1 [Myotis davidii] |
| 98 | PREDICTED: trace amine-associated receptor 1 [Marmota marmota marmota] |
| 99 | trace amine-associated receptor 1 [Marmota flaviventris] |
| 100 | PREDICTED: trace amine-associated receptor 1 [Panthera tigris altaica] |
| 101 | trace amine-associated receptor 1 [Ictidomys tridecemlineatus] |
| 102 | trace amine-associated receptor 1 [Bubalus bubalis] |
| 103 | PREDICTED: trace amine-associated receptor 1 [Orvctolagus cuniculus] |
| 104 | trace amine-associated receptor 1 [Urocitellus parryii] |
| 105 | PREDICTED: trace amine-associated receptor 1 [Capra hircus] |
| 106 | PREDICTED: trace amine-associated receptor 1 [Bos mutus] |
| 107 | trace amine-associated receptor 1 [Bos taurus] |
| 108 | PREDICTED: trace amine-associated receptor 1 [Chinchilla lanigera] |
| 109 | trace amine-associated receptor 1 isoform X1 [Neophocaena asiaeorientalis asiaeorientalis] |
| 110 | trace amine-associated receptor 1 isoform X1 [Phocoena sinus] |
| 111 | trace amine-associated receptor 1-like [Heterocephalus plaber] |
| 112 | trace amine-associated receptor 1-like [Heterocephalus plaber] |
| 113 | trace amine-associated receptor 1 [Odocoileus virginianus texanus] |
| 114 | PREDICTED: trace amine-associated receptor 1 [Condylura cristata] |
| 115 | PREDICTED: trace amine-associated receptor 1 [Erinaceus europaeus] |
| 116 | trace amine-associated receptor 1 [Fukomys damarensis] |
| 117 | trace amine-associated receptor 1 [Nannospalax galili] |
| 118 | LOW QUALITY PROTEIN: trace amine-associated receptor 1 [Castor canadensis] |
| 119 | PREDICTED: trace amine-associated receptor 1 [Sorex araneus] |
| 120 | trace amine-associated receptor 1 [Sturnira hondurensis] |
| 121 | trace amine-associated receptor 1 [Octodon degus] |
| 122 | PREDICTED: trace amine-associated receptor 1 [Jaculus jaculus] |
| 123 | PREDICTED: trace amine-associated receptor 1 [Dipodomys ordii] |
| 124 | trace amine-associated receptor 1 [Sarcophilus harrisii] |
| 125 | trace amine-associated receptor 1 [Dipodomys spectabilis] |
| 126 | trace amine-associated receptor 1 [Cavia porcellus] |
| 127 | PREDICTED: trace amine-associated receptor 1 [Monodelphis domestica] |
| 128 | trace amine-associated receptor 1-like [Trichosurus vulpecula] |
| 129 | trace amine-associated receptor 1 [Peromyscus leucopus] |
| 130 | trace amine-associated receptor 1 [Microtus ochropaster] |

**[Table 3-3]**

| | Reference gene |
|---|---|
| 131 | trace amine-associated receptor 1 [Onychomys torridus] |
| 132 | trace amine-associated receptor 1-like [Vombatus ursinus] |
| 133 | trace amine-associated receptor 1 [Peromyscus maniculatus bairdii] |
| 134 | trace amine-associated receptor 1 [Rattus norvegicus] |
| 135 | trace amine-associated receptor 1 [Rattus rattus] |
| 136 | trace amine-associated receptor 1 [Mesocricetus auratus] |
| 137 | trace amine-associated receptor 1 [Microtus oregoni] |
| 138 | trace amine-associated receptor 1 [Cricetulus griseus] |
| 139 | PREDICTED: trace amine-associated receptor 1-like isoform X1 [Crocodylus porosus] |
| 140 | trace amine-associated receptor 1 [Mus musculus] |
| 141 | trace amine-associated receptor 1 [Mus caroli] |
| 142 | LOW QUALITY PROTEIN: trace amine-associated receptor 1-like [Phascolarctos cinereus] |
| 143 | PREDICTED: trace amine-associated receptor 1-like [Gavialis gangeticus] |
| 144 | trace amine-associated receptor 1 [Alligator sinensis] |
| 145 | trace amine-associated receptor 1 [Grammomys surdaster] |
| 146 | trace amine-associated receptor 1 [Mastomys coucha] |
| 147 | PREDICTED: trace amine-associated receptor 1 isoform X2 [Alligator mississippiensis] |
| 148 | trace amine-associated receptor 1-like [Meriones unpuiculatus] |
| 149 | PREDICTED: trace amine-associated receptor 1 isoform X3 [Alligator mississippiensis] |
| 150 | PREDICTED: trace amine-associated receptor 1 isoform X1 [Alligator mississippiensis] |
| 151 | trace amine-associated receptor 1 [Mus pahari] |
| 152 | trace amine-associated receptor 1 [Arvicanthis niloticus] |
| 153 | trace amine-associated receptor 1 [Mauremys reevesii] |
| 154 | trace amine-associated receptor 1-like [Grammomys surdaster] |
| 155 | trace amine-associated receptor 1 [Arvicola amphibius] |
| 156 | trace amine-associated receptor 1 [Chelonia mydas] |
| 157 | trace amine-associated receptor 1 [Ornithorhynchus anatinus] |
| 158 | trace amine-associated receptor 1-like [Tachyplossus aculeatus] |
| 159 | trace amine-associated receptor 1 [Chrysemys picta bellii] |
| 160 | trace amine-associated receptor 1 [Terrapene carolina triunguis] |
| 161 | trace amine-associated receptor 1-like [Trachemys scripta elepans] |
| 162 | trace amine-associated receptor 1 [Gopherus evgoodei] |
| 163 | trace amine-associated receptor 1-like [Dermochelys coriacea] |
| 164 | LOW QUALITY PROTEIN: trace amine-associated receptor 1 [Chelonoidis abingdonii] |
| 165 | trace amine-associated receptor 1 [Pelodiscus sinensis] |
| 168 | trace amine-associated receptor 1 [Orycteropus afer afer] |
| 167 | PREDICTED: trace amine-associated receptor 1 [Balearica regulorum gibbericeps] |
| 168 | PREDICTED: trace amine-associated receptor 1-like [Chlamydotis macqueenii] |
| 169 | PREDICTED: trace amine-associated receptor 1 [Cariama cristata] |
| 170 | trace amine-associated receptor 1 [Athene cunicularia] |
| 171 | PREDICTED: trace amine-associated receptor 1 [Apteryx mantelli mantelli] |
| 172 | trace amine-associated receptor 1 [Apteryx rowi] |
| 173 | trace amine-associated receptor 1 [Falco cherrug] |
| 174 | PREDICTED: trace amine-associated receptor 1 [Mesitomis unicolor] |
| 175 | trace amine-associated receptor 1 [Aquila chrysaetos chrysaetos] |
| 176 | PREDICTED: trace amine-associated receptor 1-like [Fulmarus glacialis] |
| 177 | trace amine-associated receptor 1 [Falco naumanni] |
| 178 | trace amine-associated receptor 1 [Falco pereprinus] |
| 179 | trace amine-associated receptor 1-like [Rhinatrema bivittatum] |
| 180 | PREDICTED: LOW QUALITY PROTEIN: trace amine-associated receptor 1 [Nipponia nippon] |
| 181 | trace amine-associated receptor 1 [Egretta garzetta] |
| 182 | PREDICTED: trace amine-associated receptor 1 [Cuculus canorus] |
| 183 | PREDICTED: trace amine-associated receptor 1-like [Phaethon lepturus] |
| 184 | PREDICTED: trace amine-associated receptor 1-like [Merops nubicus] |
| 185 | PREDICTED: trace amine-associated receptor 1 [Haliaeetus albicilla] |
| 186 | PREDICTED: trace amine-associated receptor 1 [Charadrius vociferus] |
| 187 | PREDICTED: trace amine-associated receptor 1 [Buceros rhinoceros silvestris] |
| 188 | PREDICTED: trace amine-associated receptor 1 [Gavia stellata] |
| 189 | trace amine-associated receptor 1 [Nothoprocta perdicaria] |
| 190 | trace amine-associated receptor 1 [Tyto alba alba] |
| 191 | PREDICTED: trace amine-associated receptor 1-like [Eurypyqa helias] |
| 192 | PREDICTED: trace amine-associated receptor 1 [Leptosomus discolor] |
| 193 | PREDICTED: trace amine-associated receptor 1-like [Pyposcelis adeliae] |
| 194 | PREDICTED: trace amine-associated receptor 1-like [Tinamus puttatus] |
| 195 | trace amine-associated receptor 1-like [Pipra filicauda] |

**[Table 3-4]**

| | Reference gene |
|---|---|
| 196 | PREDICTED: trace amine-associated receptor 1-like [Lepidothrix coronata] |
| 197 | PREDICTED: trace amine-associated receptor 1-like [Tinamus guttatus] |
| 198 | PREDICTED: trace amine-associated receptor 1 [Aptenodytes forsteri] |
| 199 | trace amine-associated receptor 1 [Python bivittatus] |
| 200 | trace amine-associated receptor 1 [Pseudonaja textilis] |
| 201 | trace amine-associated receptor 1 [Antrostomus carolinensis] |
| 202 | trace amine-associated receptor 1 [Cygnus atratus] |
| 203 | trace amine-associated receptor 1 [Hirundo rustica] |
| 204 | trace amine-associated receptor 1 [Notechis scutatus] |
| 205 | trace amine-associated receptor 1 isoform X1 [Protobothrops mucrosquamatus] |
| 206 | PREDICTED: trace amine-associated receptor 1 [Pelecanus crispus] |
| 207 | trace amine-associated receptor 1-like [Empidonax traillii] |
| 208 | trace amine-associated receptor 1 [Microcaecilia unicolor] |
| 209 | trace amine-associated receptor 1 [Manacus vitellinus] |
| 210 | LOW QUALITY PROTEIN: trace amine-associated receptor 1 [Cygnus olor] |
| 211 | PREDICTED: trace amine-associated receptor 1 [Gekko japonicus] |
| 212 | trace amine-associated receptor 1-like [Neopelma chrysocephalum] |
| 213 | PREDICTED: trace amine-associated receptor 1 [Sturnus vulgaris] |
| 214 | trace amine-associated receptor 1 [Corvus moneduloides] |
| 215 | trace amine-associated receptor 1 [Gallus gallus] |
| 216 | PREDICTED: trace amine-associated receptor 1 [Anser cypnoides domesticus] |
| 217 | PREDICTED: trace amine-associated receptor 1 [Corvus brachyrhynchos] |
| 218 | trace amine-associated receptor 1 [Catharus ustulatus] |
| 219 | PREDICTED: trace amine-associated receptor 1-like [Colius striatus] |
| 220 | trace amine-associated receptor 1 [Motacilla alba alba] |
| 221 | PREDICTED: trace amine-associated receptor 1-like [Calidris pugnax] |
| 222 | trace amine-associated receptor 1 [Taeniopygia puttata] |
| 223 | trace amine-associated receptor 1 [Gallus gallus] |
| 224 | PREDICTED: trace amine-associated receptor 1 [Apaloderma vittatum] |
| 225 | trace amine-associated receptor 1 [Anas platyrhynchos] |
| 226 | trace amine-associated receptor 1 [Parus major] |
| 227 | trace amine-associated receptor 1 [Aythya fuligula] |
| 228 | trace amine-associated receptor 1-like [Geotrypetes seraphini] |
| 229 | PREDICTED: trace amine-associated receptor 1 [Thamnophis sirtalis] |
| 230 | PREDICTED: trace amine-associated receptor 1 [Pseudopodoces humilis] |
| 231 | trace amine-associated receptor 1 [Zonotrichia albicollis] |
| 232 | trace amine-associated receptor 1 [Corvus kubaryi] |
| 233 | trace amine-associated receptor 1 [Xenopus tropicalis] |
| 234 | trace amine-associated receptor 1 [Meleapris pallopavo] |
| 235 | trace amine-associated receptor 1 [Xenopus laevis] |
| 236 | PREDICTED: trace amine-associated receptor 1-like [Tauraco erythrolophusl |
| 237 | trace amine-associated receptor 1 [Molothrus ater] |
| 238 | trace amine-associated receptor 1-like [Chiroxiphia lanceolata] |
| 239 | trace amine-associated receptor 1 [Serinus canaria] |
| 240 | trace amine-associated receptor 1 [Pantherophis guttatus] |
| 241 | LOW QUALITY PROTEIN: trace amine-associated receptor 1 [Thamnophis elegans] |
| 242 | trace amine-associated receptor 1-like [Lonchura striata domestica] |
| 243 | trace amine-associated receptor 1 [Calypte anna] |
| 244 | trace amine-associated receptor 1-like [Corapipo altera] |
| 245 | trace amine-associated receptor 1 [Oxyura iamaicensis] |
| 246 | trace amine-associated receptor 1 [Phasianus colchicus] |
| 247 | trace amine-associated receptor 1 [Cotumix japonica] |
| 248 | PREDICTED: trace amine-associated receptor 1 [Anolis carolinensis] |
| 249 | PREDICTED: trace amine-associated receptor 1 [Ficedula albicollis] |
| 250 | trace amine-associated receptor 1-like [Bufo bufo] |

**[Table 4]**

| No. | GPCR | The number of reference genes | No. | GPCR | The number of reference genes | No. | GPCR | The number of reference genes |
|---|---|---|---|---|---|---|---|---|
| 1 | VN1R1 | 8 | 51 | GPR15 | 249 | 101 | GPR162 | 248 |
| 2 | VN1R2 | 32 | 52 | GPR17 | 250 | 102 | GPR171 | 250 |
| 3 | VN1R4 | 15 | 53 | GPR19 | 250 | 103 | GPR173 | 250 |
| 4 | VN1R5 | 6 | 54 | GPR20 | 250 | 104 | GPR174 | 250 |
| 5 | TAS1R1 | 250 | 55 | GPR21 | 248 | 105 | GPR176 | 141 |
| 6 | TAS1R2 | 227 | 56 | GPR22 | 250 | 106 | GPR182 | 249 |
| 7 | TAS1R3 | 250 | 57 | GPR25 | 249 | 107 | GPR183 | 250 |
| 8 | TAS2R8 | 91 | 58 | GPR26 | 250 | 108 | GPR42 | 242 |
| 9 | TAS2R16 | 142 | 59 | GPR27 | 206 | 109 | ADGRA1 | 231 |
| 10 | TAS2R38 | 156 | 60 | GPR31 | 179 | 110 | ADGRA2 | 250 |
| 11 | TAS2R42 | 180 | 61 | GPR32 | 100 | 111 | ADGRA3 | 250 |
| 12 | TAS2R45 | 20 | 62 | GPR33 | 250 | 112 | ADGRB1 | 235 |
| 13 | TAS2R46 | 71 | 63 | GPR34 | 249 | 113 | ADGRB2 | 239 |
| 14 | TAS2R31 | 37 | 64 | GPR35 | 121 | 114 | ADGRB3 | 237 |
| 15 | TAS2R1 | 206 | 65 | GPR37 | 250 | 115 | ADGRC1 | 242 |
| 16 | TAS2R3 | 183 | 66 | GPR37L1 | 162 | 116 | ADGRC2 | 250 |
| 17 | TAS2R4 | 195 | 67 | GPR39 | 250 | 117 | ADGRC3 | 250 |
| 18 | TAS2R5 | 123 | 68 | GPR45 | 234 | 118 | ADGRD1 | 242 |
| 19 | TAS2R7 | 167 | 69 | GPR48 | 250 | 119 | ADGRD2 | 250 |
| 20 | TAS2R9 | 95 | 70 | GPR49 | 250 | 120 | ADGRE1 | 232 |
| 21 | TAS2R10 | 202 | 71 | GPR50 | 227 | 121 | ADGRE2 | 38 |
| 22 | TAS2R13 | 86 | 72 | GPR52 | 250 | 122 | ADGRE3 | 238 |
| 23 | TAS2R14 | 110 | 73 | GPR61 | 250 | 123 | ADGRE5 | 250 |
| 24 | TAS2R20 | 37 | 74 | GPR62 | 177 | 124 | ADGRFI | 236 |
| 25 | TAS2R30 | 7 | 75 | GPR63 | 250 | 125 | ADGRF3 | 250 |
| 26 | TAS2R39 | 142 | 76 | GPR65 | 250 | 126 | ADGRF4 | 250 |
| 27 | TAS2R40 | 157 | 77 | GPR68 | 250 | 127 | ADGRF5 | 250 |
| 28 | TAS2R43 | 33 | 78 | GPR75 | 249 | 128 | ADGRG1 | 217 |
| 29 | TAS2R50 | 30 | 79 | GPR78 | 100 | 129 | ADGRG2 | 250 |
| 30 | TAS2R60 | 130 | 80 | GPR82 | 249 | 130 | ADGRG4 | 250 |
| 31 | TAAR1 | 250 | 81 | GPR83 | 248 | 131 | ADGRG5 | 250 |
| 32 | TAAR2 | 191 | 82 | GPR84 | 249 | 132 | ADGRG6 | 250 |
| 33 | TAAR5 | 223 | 83 | GPR85 | 89 | 133 | ADGRG7 | 233 |
| 34 | TAAR8 | 120 | 84 | GPR87 | 250 | 134 | ADGRL1 | 232 |
| 35 | TAAR9 | 138 | 85 | GPR88 | 249 | 135 | ADGRL2 | 250 |
| 36 | TAAR6 | 160 | 86 | GPR101 | 250 | 136 | ADGRL3 | 250 |
| 37 | MrgprE | 73 | 87 | GPR132 | 250 | 137 | ADGRL4 | 250 |
| 38 | MrgprF | 182 | 88 | GPR135 | 250 | 138 | Chrm-4/M4R | 244 |
| 39 | MAS1 | 26 | 89 | GPR139 | 250 | 139 | F2RL1 | 248 |
| 40 | MAS1L | 18 | 90 | GPR141 | 250 | 140 | GRM5 | 250 |
| 41 | MRGPRD | 156 | 91 | GPR142 | 250 | 141 | APLNR | 250 |
| 42 | MRGPRG | 169 | 92 | GPR146 | 250 | 142 | CALCRL | 250 |
| 43 | MRGPRX1 | 48 | 93 | GPR148 | 249 | 143 | GLP2R | 250 |
| 44 | MRGPRX2 | 176 | 94 | GPR149 | 239 | 144 | MC4R | 249 |
| 45 | MRGPRX3 | 38 | 95 | GPR150 | 247 | 145 | CCR6 | 250 |
| 46 | MRGPRX4 | 10 | 96 | GPR151 | 250 | | | |
| 47 | GPR3 | 235 | 97 | GPR152 | 217 | | | |
| 48 | GPR4 | 250 | 98 | GPR153 | 250 | | | |
| 49 | GPR6 | 250 | 99 | GPR160 | 250 | | | |
| 50 | GPR12 | 250 | 100 | GPR161 | 249 | | | |

As for the GPCRs of Nos. 1 to 145 in Table 4, the alignment analysis on the identified gene group was conducted using Clustal W. On the basis of the alignment results, consensus receptor design was performed using Jalview. In the alignment, when one amino acid residue which was different from the amino acid residue in a reference amino acid sequence and had a frequency of appearance of 50% or more existed at a position corresponding to each amino acid position in the amino acid sequence of the original receptor of the receptor of interest serving as the reference, the amino acid residue in the reference amino acid sequence was altered to the amino acid residue. Even in the case where one amino acid residue which was different from the amino acid residue in a reference amino acid sequence and had a frequency of appearance of 50% existed at a position corresponding to each amino acid position in the amino acid sequence of the original receptor of the receptor of interest serving as the reference, the amino acid residue of the reference amino acid sequence was not altered when the amino acid residue in the reference amino acid sequence also had a frequency of appearance of 50%. In the alignment, when deletion existed with a frequency of appearance of 40% or more at a position corresponding to each amino acid position in the amino acid sequence of the original receptor of the receptor of interest serving as the reference, the amino acid residue in the reference amino acid sequence was altered to be deleted. For example, since deletion existed with a frequency of appearance of 40% or more at a position corresponding to a C-terminal amino acid position, as in TAAR1, the amino acid residue in the reference amino acid sequence was altered to be deleted. Further, for example, since deletion was found with a frequency of appearance of 40% or more at a position corresponding to an initiation methionine position in the human sequence, as in TAAR1, TAAR2, MAS1L, GPR135, and ADGRG4, the amino acid residue in the reference amino acid sequence was altered to be deleted. Further the first methionine in the amino acid sequence thus altered by the procedures described above was selected as an initiation methionine, and an amino acid sequence upstream thereof was deleted. However, in this method, as for TAAR6, when a consensus residue on an N-terminal side of a consensus residue consisting of a methionine residue positioned nearest to the N terminus was changed to the absence of a consensus residue, the full-length of the amino acid sequence of the resulting consensus receptor was shorter by 10% or more than the full-length of the amino acid sequence of the original receptor. Hence, an N-terminal structure of the original receptor was maintained as it was instead of following this method. Specifically, since the consensus residue asparagine nearest to the N terminus was an amino acid residue important for sugar chain modification and membrane translocation, the consensus residue was not changed, and an N-terminal structure on an N-terminal side of a position corresponding to the consensus residue in the original receptor was maintained as it was. On the other hand, in the alignment, when an amino acid existed with a frequency of appearance of 60% or more at a position corresponding to a deletion position in the amino acid sequence of the original receptor of the receptor of interest serving as the reference, the deletion position in the reference amino acid sequence was altered so as to insert the most highly conservative amino acid at this position. When two or more amino acids were most highly conservative, the position was altered so as to insert an amino acid having the smallest molecular weight at this position. For example, since an amino acid existed with a frequency of appearance of 60% or more on an N-terminal side of a position corresponding to an N-terminal amino acid position, as in TAS2R1, an amino acid residue was added to an N-terminal side of the N terminus of the reference amino acid sequence.

Receptor topology in the design was confirmed using TMHMM (Transmembrane Hidden Markov Model). The various receptors thus designed are required to have a seven-transmembrane structure.

As for the GPCRs of Nos. 1 to 5, 7 to 13, 31 to 38, 60, 76, 80, 85, and 102 in Table 4, DNA sequences encoding various receptor polypeptides thus designed were obtained by DNA synthesis after optimization of nucleotide sequence codons corresponding to the amino acid sequences for expressions in human cultured cells. EcoRI and XhoI sites were added to both ends of the nucleotide sequence and recombined with EcoRI and XhoI sites prepared downstream of a Flag-Rho tag sequence in a pME18S vector. A gene encoding human RTP1S for translocation of a GPCR protein produced in cultured cells onto cell membranes was incorporated into EcoRI and XhoI sites of another pME18S vector to prepare a pME18S-RTP1S vector.

### 2) Preparation 1 of cultured cell transformed with receptor gene

For flow cytometry, 3.3 × 10⁵ HEK293 cells suspended in DMEM (Nacalai) were inoculated to each well of a 6-well dish, and 24 hours later, a reaction solution was prepared according to the composition shown in Table 5, left standing for 20 minutes in a clean bench, and then added to each well of the 6-well dish. In this context, the receptor genes refer to the genes of the GPCRs of Nos. 1 to 4, 8 to 13, 31 to 38, 60, 76, 80, 85, and 102 in Table 4. The cells were cultured for 24 hours in an incubator kept at 37°C and 5% CO₂. Cells that expresses no receptor (mock) were provided as a control.

**[Table 5]**

| | |
|---|---|
| DMEM (Nacalai) | 100 µL |
| Receptor gene (incorporated into pME18S vector) | 3.0 µg |
| pME18S-RTP1S | 1.0 µg |
| Polyethylenimine-MAX (0.1% Aqueous solution (pH 7.4), COSMO BIO) | 10 µL |

### 3) Preparation 2 of cultured cell transformed with receptor gene

For flow cytometry, 3.3 × 10⁵ HEK293 cells suspended in DMEM (Nacalai) were inoculated to each well of a 6-well dish, and 24 hours later, a reaction solution was prepared according to the composition shown in Table 6, left standing for 20 minutes in a clean bench, and then added to each well of the 6-well dish. The cells were cultured for 24 hours in an incubator kept at 37°C and 5% CO₂. Cells expressing no receptor (Mock) were provided as a control.

**[Table 6]**

| | |
|---|---|
| DMEM (Nacalai) | 100 µL |
| FLAG-TAS1 R1 or FLAG-cTAS1R1 (incorporated into pME18S vector) | 2.0 µg |
| TAS1R3 or cTAS1R3 (incorporated into pME18S vector) | 2.0 µg |
| Polyethylenimine-MAX (0.1% Aqueous solution (pH 7.4), COSMO BIO) | 10 µL |
| *"c" indicates consensus. | |

### 4) Measurement of receptor protein level on cell membrane (flow cytometry)

An anti-FLAG antibody (Cosmo Bio Co., Ltd.) was allowed to act as a primary antibody on cells recovered using a cell stripper on ice for 1 hour. After washing of the cells, PE (phycoerythrin)-conjugated anti-mouse IgG (Abcam plc.) was allowed to act thereon as a secondary antibody on ice for 30 minutes. After washing, 0.25 µg of 7-AAD (7-aminoactinomycin D, FUJIFILM Wako Pure Chemical Corp.) was added thereto, and a mean of PE signals of a 7-AAD-negative cell population was measured as an index for a receptor level on cell membranes using a flow cytometry system (Becton, Dickinson and Company). In each experiment run, a mean of PE signals was determined in the same manner as above by using cells expressing a FLAG-M2 acetylcholine receptor as a positive control (PC) and cells expressing no receptor as a negative control (NC). Normalization was performed with the NC PE signal defined as 0% and the PC PE signal defined as 100%. The PE signals (%) of various receptors were calculated as membrane expression levels (Cell surface expression).

### 5) Results

Using flow cytometry (2) and 4) above), the expression level of each receptor on HEK293 cells was analyzed. As shown in Table 7, as a result of comparing the average values of three experiments, increase in membrane expression level by the consensus design method was found for all the receptors whose membrane expression levels had been analyzed, specifically VN1R1, VN1R2, VN1R4, VN1R5, TAS2R8, TAS2R16, TAS2R38, TAS2R42, TAS2R45, TAS2R46, TAAR1, TAAR2, TAAR5, TAAR6, TAAR8, TAAR9, MrgprE, MrgprF, GPR31, GPR65, GPR82, GPR88, and GPR171.

**[Table 7]**

| | Original | | Consensus | |
|---|---|---|---|---|
| | Membrane expression level (%) | SEM | Membrane expression level (%) | SEM |
| VN1R1 | -0.05 | 0.03 | 4.17 | 1.07 |
| VN1R2 | -0.05 | 0.03 | 14.42 | 1.29 |
| VN1R4 | 0.37 | 0.14 | 0.87 | 0.00 |
| VN1R5 | 0.18 | 0.04 | 0.31 | 0.03 |
| TAS2R8 | 28.58 | 1.26 | 53.59 | 1.88 |
| TAS2R16 | 36.00 | 0.64 | 49.31 | 1.86 |
| TAS2R38 | 2.63 | 0.13 | 14.21 | 0.61 |
| TAS2R42 | 1.66 | 0.09 | 2.95 | 0.05 |
| TAS2R45 | 0.17 | 0.03 | 0.37 | 0.10 |
| TAS2R46 | 2.83 | 0.17 | 16.38 | 0.85 |
| TAAR1 | 54.87 | 0.72 | 90.97 | 3.56 |
| TAAR2 | 0.13 | 0.08 | 0.25 | 0.04 |
| TAAR5 | 0.05 | 0.00 | 34.52 | 1.65 |
| TAAR8 | 0.06 | 0.01 | 5.54 | 0.26 |
| TAAR9 | 0.25 | 0.02 | 5.31 | 0.16 |
| TAAR6 | 0.10 | 0.01 | 21.82 | 0.82 |
| MrgprE | 18.59 | 0.92 | 27.41 | 2.25 |
| MrgprF | 95.21 | 12.63 | 136.39 | 9.09 |
| GPR31 | 55.21 | 10.07 | 112.78 | 17.43 |
| GPR65 | 39.00 | 6.41 | 59.40 | 9.67 |
| GPR82 | 99.43 | 16.91 | 113.22 | 17.61 |
| GPR88 | 7.25 | 1.18 | 27.25 | 4.51 |
| GPR171 | 91.17 | 14.12 | 108.04 | 15.93 |

Using flow cytometry (3) and 4) above), the expression level of a combination of the original receptor of TAS1R1 and the original receptor of TAS1R3, the consensus receptor of TAS1R1 and the consensus receptor of TAS1R3, the original receptor of TAS1R1 and the consensus receptor of TAS1R3, or the consensus receptor of TAS1R1 and the original receptor of TAS1R3 on HEK293 cells was analyzed. As shown in Figure 1, it was found that in comparison with when the two original receptors of TAS1R1 and TAS1R3 were co-expressed on HEK293 cells, when any of the consensus receptors was expressed, the membrane expression level of TAS1R1 was increased. In particular, the most abundant expression was observed in the case of the consensus design of both TAS1R1 and TAS1R3.

Tables 8-1 and 8-2 below show the Accession Nos. and amino acid sequences of original GPCRs and the amino acid sequences of consensus GPCRs in the above Examples. Further, SEQ ID NOs: 215 to 234 and 313 to 317 show DNA sequences encoding consensus GPCRs consisting of amino acid sequences of SEQ ID NOs: 108 to 112, 114 to 120, 138 to 145, 167, 183, 187, 192, and 209.

**[Table 8-1]**

| No. | GPCR | Accession No. | SEQ ID NO of amino acid sequence | SEQ ID NO of consensus amino acid sequence | No. | GPCR | Accession No. | SEQ ID NO of amino acid sequence | SEQ ID NO of consensus amino acid sequence |
|---|---|---|---|---|---|---|---|---|---|
| 1 | VN1R1 | NP_065684.1 | 1 | 108 | 55 | GPR21 | NP_005285.1 | 55 | 162 |
| 2 | VN1R2 | NP_7762550.2 | 2 | 109 | 56 | GPR22 | NP_005286.2 | 56 | 163 |
| 3 | VN1R4 | NP_776256.2 | 3 | 110 | 57 | GPR25 | NP_005289.2 | 57 | 164 |
| 4 | VN1R5 | NP_776257.1 | 4 | 111 | 58 | GPR26 | NP_703143.1 | 58 | 165 |
| 5 | TAS1R1 | NP_619642.2 | 5 | 112 | 59 | GPR27 | NP_061844.1 | 59 | 166 |
| 6 | TAS1R2 | NP 689418.2 | 6 | 113 | 60 | GPR31 | NP_005290.2 | 60 | 167 |
| 7 | TAS1R3 | NP_689414.2 | 1 | 114 | 61 | GPR32 | NP_001497.1 | 61 | 168 |
| 8 | TAS2R8 | NP_076407.1 | 8 | 115 | 62 | GPR33 | NP_001184113.2 | 62 | 169 |
| 9 | TAS2R16 | NP 058641.1 | 9 | 116 | 63 | GPR34 | NP_001091048.1 | 63 | 170 |
| 10 | TAS2R38 | NP_789787.5 | 10 | 117 | 64 | GPR35 | NP_001182310.1 | 64 | 171 |
| 11 | TAS2R42 | NP_852094.2 | 11 | 118 | 65 | GPR37 | NP_005293.1 | 65 | 172 |
| 12 | TAS2R45 | NP_795367.2 | 12 | 119 | 66 | GPR37L1 | NP_004758.3 | 66 | 173 |
| 13 | TAS2R46 | NP_795368.2 | 13 | 120 | 67 | GPR39 | NP_001499.1 | 67 | 174 |
| 14 | TAS2R31 | NP_795366.2 | 14 | 121 | 68 | GPR45 | NP_009158.3 | 68 | 175 |
| 15 | TAS2R1 | NP_001373277.1 | 15 | 122 | 69 | GPR48 | NP_001333361.1 | 69 | 176 |
| 16 | TAS2R3 | NP_058639.1 | 16 | 123 | 70 | GPR49 | NP_003658.1 | 70 | 177 |
| 17 | TAS2R4 | NP_058640.1 | 17 | 124 | 71 | GPR50 | NP_004215.2 | 71 | 178 |
| 18 | TAS2R5 | NP_061853.1 | 18 | 125 | 72 | GPR52 | NP_005675.3 | 72 | 179 |
| 19 | TAS2R7 | NP_076408.1 | 19 | 126 | 73 | GPR61 | NP_001380836.1 | 73 | 180 |
| 20 | TAS2R9 | NP_076406.1 | 20 | 127 | 74 | GPR62 | NP_543141.3 | 74 | 181 |
| 21 | TAS2R10 | NP_076410.1 | 21 | 128 | 75 | GPR63 | NP_001137429.1 | 75 | 182 |
| 22 | TAS2R13 | NP_076409.1 | 22 | 129 | 76 | GPR65 | NP_003599.2 | 76 | 183 |
| 23 | TAS2R14 | NP_076411.1 | 23 | 130 | 77 | GPR68 | XP_005268167.1 | 77 | 184 |
| 24 | TAS2R20 | NP_795370.2 | 24 | 131 | 78 | GPR75 | NP_006785.1 | 78 | 185 |
| 25 | TAS2R30 | NP_001091112.1 | 25 | 132 | 79 | GPR78 | NP_543009.2 | 79 | 186 |
| 26 | TAS2R39 | NP_795362.2 | 26 | 133 | 80 | GPR82 | NP_543007.1 | 80 | 187 |
| 27 | TAS2R40 | NP_795363.1 | 27 | 134 | 81 | GPR83 | NP_001317274.1 | 81 | 188 |
| 28 | TAS2R43 | NP_795365.2 | 28 | 135 | 82 | GPR84 | NP_065103.1 | 82 | 189 |
| 29 | TAS2R50 | NP_795371.2 | 29 | 136 | 83 | GPR85 | NP_001139737.1 | 83 | 190 |
| 30 | TAS2R60 | NP 803186.1 | 30 | 137 | 84 | GPR87 | NP_076404.3 | 84 | 191 |
| 31 | TAAR1 | NP_612200.1 | 31 | 138 | 85 | GPR88 | NP_071332.2 | 85 | 192 |
| 32 | TAAR2 | NP_001028252.1 | 32 | 139 | 86 | GPR101 | NP_473362.1 | 86 | 193 |
| 33 | TAARS | NP 003958.2 | 33 | 140 | 87 | GPR132 | NP_001265623.1 | 87 | 194 |
| 34 | TAAR8 | NP_444508.1 | 34 | 141 | 88 | GPR135 | NP_072093.2 | 88 | 195 |
| 35 | TAAR9 | NP_778227.3 | 35 | 142 | 89 | GPR139 | NP_001002911.1 | 89 | 196 |
| 36 | TAAR6 | NP_778237.1 | 36 | 143 | 90 | GPR141 | NP_001316922.1 | 90 | 197 |
| 37 | MrgprE | NP_001034254.2 | 37 | 144 | 91 | GPR142 | NP_001318005.1 | 91 | 198 |
| 38 | MrgprF | NP_001091985.1 | 38 | 145 | 92 | GPR146 | NP_001290402.1 | 92 | 199 |
| 39 | MAS1 | NP_001353633.1 | 39 | 146 | 93 | GPR148 | NP_997247.2 | 93 | 200 |
| 40 | MAS1L | NP_4431991 | 40 | 147 | 94 | GPR149 | NP_001033794.1 | 94 | 201 |
| 41 | MRGPRD | NP_9446052 | 41 | 148 | 95 | GPR150 | NP_954713.1 | 95 | 202 |
| 42 | MRGPRG | NP_001157849.1 | 42 | 149 | 96 | GPR151 | NP_919227.2 | 96 | 203 |
| 43 | MRGPRX1 | NP_001380507.1 | 43 | 150 | 97 | GPR152 | NP_996880.1 | 97 | 204 |
| 44 | MRGPRX2 | NP_0012905441 | 44 | 151 | 98 | GPR153 | NP_997253.2 | 98 | 205 |
| 45 | MRGPRX3 | NP_001357393.1 | 45 | 152 | 99 | GPR160 | NP_055188.1 | 99 | 206 |
| 46 | MRGPRX4 | NP_473373.2 | 46 | 153 | 100 | GPR161 | NP_001254538.1 | 100 | 207 |
| 47 | GPR3 | NP_005272.1 | 47 | 154 | 101 | GPR162 | NP_055264.1 | 101 | 208 |
| 48 | GPR4 | NP_005273.1 | 48 | 155 | 102 | GPR171 | NP_037440.3 | 102 | 209 |
| 49 | GPR6 | NP_001273028.1 | 49 | 156 | 103 | GPR173 | NP_061842.1 | 103 | 210 |
| 50 | GPR12 | NP_005279.1 | 50 | 157 | 104 | GPR174 | NP_115942.1 | 104 | 211 |
| 51 | GPR15 | NP_005281.1 | 51 | 158 | 105 | GPR176 | NP_001258783.1 | 105 | 212 |
| 52 | GPR17 | NP_001154887.1 | 52 | 159 | 106 | GPR182 | NP_009195.1 | 108 | 213 |
| 53 | GPR19 | NP_006134.2 | 53 | 160 | 107 | GPR183 | NP_004942.1 | 107 | 214 |
| 54 | GPR20 | NP_005284.2 | 54 | 161 | | | | | |

**[Table 8-2]**

| No. | GPCR | Accession No. | SEQ ID NO of amino acid sequence | SEQ ID NO of consensus amino acid sequence | No. | GPCR | Accession No. | SEQ ID NO of amino acid sequence | SEQ ID NO of consensus amino acid sequence |
|---|---|---|---|---|---|---|---|---|---|
| 108 | GPR42 | NP_001335124.1 | 237 | 275 | 127 | ADGRF5 | NP_001091988.1 | 256 | 294 |
| 109 | ADGRA1 | NP_001077378.1 | 238 | 276 | 128 | ADGRG1 | NP_001357368.1 | 257 | 295 |
| 110 | ADGRA2 | NP_116166.9 | 239 | 277 | 129 | ADGRG2 | NP_001073327.1 | 258 | 296 |
| 111 | ADGRA3 | XP_005248194.1 | 240 | 278 | 130 | ADGRG4 | NP_722576.3 | 259 | 297 |
| 112 | ADGRB1 | NP_001693.2 | 241 | 279 | 131 | ADGRG5 | NP_001291305.1 | 260 | 298 |
| 113 | ADGRB2 | NP_001281264.1 | 242 | 280 | 132 | ADGRG6 | NP_001027566.2 | 261 | 299 |
| 114 | ADGRB3 | NP_001695.2 | 243 | 281 | 133 | ADGRG7 | NP_001295291.1 | 262 | 300 |
| 115 | ADGRC1 | NP_001365257.1 | 244 | 282 | 134 | ADGRL1 | NP_001008701.1 | 263 | 301 |
| 116 | ADGRC2 | NP_001399.1 | 245 | 283 | 135 | ADGRL2 | NP_001284633.1 | 264 | 302 |
| 117 | ADGRC3 | NP_001398 | 246 | 284 | 136 | ADGRL3 | NP_001309175.1 | 265 | 303 |
| 118 | ADGRD1 | NP_001317426.1 | 247 | 285 | 137 | ADGRL4 | NP_071442.2 | 266 | 304 |
| 119 | ADGRD2 | NP_001382354.1 | 248 | 286 | 138 | Chrm-4/M4R | NP_000732.2 | 267 | 305 |
| 120 | ADGRE1 | NP_001243181.1 | 249 | 287 | 139 | F2RL1 | NP_005233.4 | 268 | 306 |
| 121 | ADGRE2 | NP_001257981.1 | 250 | 288 | 140 | GRM5 | NP_001137303.1 | 269 | 307 |
| 122 | ADGRE3 | NP_001276087.1 | 251 | 289 | 141 | APLNR | NP_005152.1 | 270 | 308 |
| 123 | ADGRE5 | NP_001020331.1 | 252 | 290 | 142 | CALCRL | NP_001258680.1 | 271 | 309 |
| 124 | ADGRF1 | NP_722582.2 | 253 | 291 | 143 | GLP2R | NP_004237.1 | 272 | 310 |
| 125 | ADGRF3 | NP_001138640.1 | 254 | 292 | 144 | MC4R | NP_005903.2 | 273 | 311 |
| 126 | ADGRF4 | NP_001334784.1 | 255 | 293 | 145 | CCR6 | NP_001381511.1 | 274 | 312 |

## Claims

1. A method for expressing a G protein-coupled receptor (GPCR) polypeptide, comprising:
expressing, in a cell, a GPCR polypeptide consisting of an amino acid sequence obtained by, in an amino acid sequence of a GPCR of interest (provided that an olfactory receptor is excluded), altering at least one amino acid residue different from that in a consensus amino acid sequence to an amino acid residue at a position corresponding thereto in the consensus amino acid sequence, wherein
the consensus amino acid sequence is an amino acid sequence derived by alignment of the amino acid sequence of the GPCR of interest and amino acid sequences of GPCRs encoded by orthologs of the GPCR of interest in vertebrates.

2. The method according to claim 1, wherein the consensus amino acid sequence is an amino acid sequence comprising consensus residues identified in accordance with the following criteria (i) to (iii) from the alignment:
(i) at each amino acid position of the alignment,
(i-i) when there exists one amino acid residue which is different from the amino acid residue of the GPCR of interest and has a frequency of appearance of 50% or more, the amino acid residue is identified as a consensus residue,
(i-ii) when there exist two amino acid residues having a frequency of appearance of 50%, the amino acid residue of the GPCR of interest is identified as a consensus residue,
(i-iii) when there exists an amino acid residue in the GPCR of interest and there exists no amino acid residue having a frequency of appearance of 40% or more, the absence of a consensus residue is identified,
(i-iv) when there exists no amino acid residue in the GPCR of interest and there exists an amino acid residue having a frequency of appearance of 60% or more, an amino acid residue having the highest frequency of appearance is identified as a consensus residue, and when there exist two or more amino acid residues having the highest frequency of appearance, an amino acid residue having the smallest molecular weight among the amino acid residues is identified as a consensus residue, and
(i-v) if none of the above (i-i) to (i-iv) is appropriate, the amino acid residue of the GPCR of interest is identified as a consensus residue;
(ii) when the consensus residues are identified in accordance with the criterion (i) and when a consensus residue nearest to the N terminus is a consensus residue at a position corresponding to the N terminus of the GPCR of interest or a C-terminal side thereof and is not a methionine residue, a consensus residue on an N-terminal side of a consensus residue consisting of a methionine residue positioned nearest to the N terminus is changed to the absence of a consensus residue; and
(iii) when the consensus residues are identified in accordance with the criterion (i) and when a consensus residue nearest to the N terminus is a consensus residue at a position corresponding to an N-terminal side of the N terminus of the GPCR of interest and is not a methionine residue, an amino acid residue having the highest frequency of appearance is identified as a consensus residue by tracing back one by one amino acid positions on an N-terminal side of the position of the consensus residue of the alignment until a methionine residue appears, and when there exist two or more amino acid residues having the highest frequency of appearance, an amino acid residue having the smallest molecular weight among the amino acid residues is identified as a consensus residue.

3. The method according to claim 1 or 2, wherein the GPCR of interest is a human GPCR.

4. The method according to any one of claims 1 to 3, wherein the GPCR polypeptide consists of an amino acid sequence obtained by, in an amino acid sequence of sequence identification number (2) of a GPCR (1) in the following Tables 1 and 2, altering at least one amino acid residue different from that in a consensus amino acid sequence of sequence identification number (3) to an amino acid residue at a position corresponding thereto in the consensus amino acid sequence:
**[Table 1]**
| No. | (1) GPCR | (2) SEQ ID NO of amino acid sequence | (3) SEQ ID NO of consensus amino acid sequence | No. | (1) GPCR | (2) SEQ ID NO of amino acid sequence | (3) SEQ ID NO of consensus amino acid sequence |
|---|---|---|---|---|---|---|---|
| | | | | | | | |
| 1 | VN1R1 | 1 | 108 | 55 | GPR21 | 55 | 162 |
| 2 | VN1R2 | 2 | 109 | 56 | GPR22 | 56 | 163 |
| 3 | VN1R4 | 3 | 110 | 57 | GPR25 | 57 | 164 |
| 4 | VN1R5 | 4 | 111 | 58 | GPR26 | 58 | 165 |
| 5 | TAS1R1 | 5 | 112 | 59 | GPR27 | 59 | 168 |
| 6 | TAS1R2 | 6 | 113 | 60 | GPR31 | 60 | 167 |
| 7 | TAS1R3 | 7 | 114 | 61 | GPR32 | 61 | 168 |
| 8 | TAS2R8 | 8 | 115 | 62 | GPR33 | 62 | 169 |
| 9 | TAS2R16 | 9 | 116 | 63 | GPR34 | 63 | 170 |
| 10 | TAS2R38 | 10 | 117 | 64 | GPR35 | 64 | 171 |
| 11 | TAS2R42 | 11 | 118 | 65 | GPR37 | 65 | 172 |
| 12 | TAS2R45 | 12 | 119 | 66 | GPR37L1 | 66 | 173 |
| 13 | TAS2R46 | 13 | 120 | 67 | GPR39 | 67 | 174 |
| 14 | TAS2R31 | 14 | 121 | 68 | GPR45 | 68 | 175 |
| 15 | TAS2R1 | 15 | 122 | 69 | GPR48 | 69 | 176 |
| 16 | TAS2R3 | 16 | 123 | 70 | GPR49 | 70 | 177 |
| 17 | TAS2R4 | 17 | 124 | 71 | GPR50 | 71 | 178 |
| 18 | TAS2R5 | 18 | 125 | 72 | GPR52 | 72 | 179 |
| 19 | TAS2R7 | 19 | 126 | 73 | GPR61 | 73 | 180 |
| 20 | TAS2R9 | 20 | 127 | 74 | GPR62 | 74 | 181 |
| 21 | TAS2R10 | 21 | 128 | 75 | GPR63 | 75 | 182 |
| 22 | TAS2R13 | 22 | 129 | 76 | GPR65 | 76 | 183 |
| 23 | TAS2R14 | 23 | 130 | 77 | GPR68 | 77 | 184 |
| 24 | TAS2R20 | 24 | 131 | 78 | GPR75 | 78 | 185 |
| 25 | TAS2R30 | 25 | 132 | 79 | GPR78 | 79 | 186 |
| 26 | TAS2R39 | 26 | 133 | 80 | GPR82 | 80 | 187 |
| 27 | TAS2R40 | 27 | 134 | 81 | GPR83 | 81 | 188 |
| 28 | TAS2R43 | 28 | 135 | 82 | GPR84 | 82 | 189 |
| 29 | TAS2R50 | 29 | 136 | 83 | GPR85 | 83 | 190 |
| 30 | TAS2R60 | 30 | 137 | 84 | GPR87 | 84 | 191 |
| 31 | TAAR1 | 31 | 138 | 85 | GPR88 | 85 | 192 |
| 32 | TAAR2 | 32 | 139 | 86 | GPR101 | 86 | 193 |
| 33 | TAAR5 | 33 | 140 | 87 | GPR132 | 87 | 194 |
| 34 | TAAR8 | 34 | 141 | 88 | GPR135 | 88 | 195 |
| 35 | TAAR9 | 35 | 142 | 89 | GPR139 | 89 | 196 |
| 37 | MrgprE | 37 | 144 | 90 | GPR141 | 90 | 197 |
| 38 | MrgprF | 38 | 145 | 91 | GPR142 | 91 | 198 |
| 39 | MAS1 | 39 | 146 | 92 | GPR146 | 92 | 199 |
| 40 | MAS1L | 40 | 147 | 93 | GPR148 | 93 | 200 |
| 41 | MRGPRD | 41 | 148 | 94 | GPR149 | 94 | 201 |
| 42 | MRGPRG | 42 | 149 | 95 | GPR150 | 95 | 202 |
| 43 | MRGPRX1 | 43 | 150 | 96 | GPR151 | 96 | 203 |
| 44 | MRGPRX2 | 44 | 151 | 97 | GPR152 | 97 | 204 |
| 45 | MRGPRX3 | 45 | 152 | 98 | GPR153 | 98 | 205 |
| 46 | MRGPRX4 | 46 | 153 | 99 | GPR160 | 99 | 206 |
| 47 | GPR3 | 47 | 154 | 100 | GPR161 | 100 | 207 |
| 48 | GPR4 | 48 | 155 | 101 | GPR162 | 101 | 208 |
| 49 | GPR6 | 49 | 156 | 102 | GPR171 | 102 | 209 |
| 50 | GPR12 | 50 | 157 | 103 | GPR173 | 103 | 210 |
| 51 | GPR15 | 51 | 158 | 104 | GPR174 | 104 | 211 |
| 52 | GPR17 | 52 | 159 | 105 | GPR176 | 105 | 212 |
| 53 | GPR19 | 53 | 160 | 106 | GPR182 | 106 | 213 |
| 54 | GPR20 | 54 | 161 | 107 | GPR183 | 107 | 214 |
**[Table 2]**
| No. | (1) GPCR | (2) SEQ ID NO of amino acid sequence | (3) SEQ ID NO of consensus amino acid sequence | No. | (1) GPCR | (2) SEQ ID NO of amino acid sequence | (3) SEQ ID NO of consensus amino acid sequence |
|---|---|---|---|---|---|---|---|
| 108 | GPR42 | 237 | 275 | 127 | ADGRF5 | 256 | 294 |
| 109 | ADGRA1 | 238 | 276 | 128 | ADGRG1 | 257 | 295 |
| 110 | ADGRA2 | 239 | 277 | 129 | ADGRG2 | 258 | 296 |
| 111 | ADGRA3 | 240 | 278 | 130 | ADGRG4 | 259 | 297 |
| 112 | ADGRB1 | 241 | 279 | 131 | ADGRG5 | 260 | 298 |
| 113 | ADGRB2 | 242 | 280 | 132 | ADGRG6 | 261 | 299 |
| 114 | ADGRB3 | 243 | 281 | 133 | ADGRG7 | 262 | 300 |
| 115 | ADGRC1 | 244 | 282 | 134 | ADGRL1 | 263 | 301 |
| 116 | ADGRC2 | 245 | 283 | 135 | ADGRL2 | 264 | 302 |
| 117 | ADGRC3 | 246 | 284 | 136 | ADGRL3 | 265 | 303 |
| 118 | ADGRD1 | 247 | 285 | 137 | ADGRL4 | 266 | 304 |
| 119 | ADGRD2 | 248 | 286 | 138 | Chrm-4/M4R | 267 | 305 |
| 120 | ADGRE1 | 249 | 287 | 139 | F2RL1 | 268 | 306 |
| 121 | ADGRE2 | 250 | 288 | 140 | GRM5 | 289 | 307 |
| 122 | ADGRE3 | 251 | 289 | 141 | APLNR | 270 | 308 |
| 123 | ADGRE5 | 252 | 290 | 142 | CALCRL | 271 | 309 |
| 124 | ADGRF1 | 253 | 291 | 143 | GLP2R | 272 | 310 |
| 125 | ADGRF3 | 254 | 292 | 144 | MC4R | 273 | 311 |
| 126 | ADGRF4 | 255 | 293 | 145 | CCR6 | 274 | 312 |

5. The method according to claim 4, wherein the GPCR polypeptide consists of an amino acid sequence of any of SEQ ID NOs: 108 to 142, 144 to 214, and 275 to 312.

6. A method for expressing a GPCR polypeptide, comprising:
expressing in a cell a GPCR polypeptide consisting of an amino acid sequence obtained by, in an amino acid sequence of a GPCR of interest, altering at least one amino acid residue different from that in a consensus amino acid sequence to an amino acid residue at a position corresponding thereto in the consensus amino acid sequence, wherein
the GPCR polypeptide consists of an amino acid sequence obtained by, in the amino acid sequence of SEQ ID NO: 36 of human TAAR6, altering at least one amino acid residue different from that in the consensus amino acid sequence of SEQ ID NO: 143 to an amino acid residue at a position corresponding thereto in the consensus amino acid sequence.

7. The method according to claim 6, wherein the GPCR polypeptide consists of the amino acid sequence of SEQ ID NO: 143.

8. A method for measuring a response of a GPCR of interest, comprising:
measuring a response of the GPCR polypeptide expressed by the method according to any one of claims 1 to 7.

9. A method for searching for a ligand for a GPCR of interest, comprising:
measuring a response of the GPCR polypeptide expressed by the method according to any one of claims 1 to 7 in the presence of a test substance; and
selecting a test substance to which the GPCR polypeptide has responded.

10. A method for evaluating and/or selecting a control agent for recognition of a ligand for a GPCR of interest, comprising:
adding a test substance and a ligand for a GPCR of interest to the GPCR polypeptide expressed by the method according to any one of claims 1 to 7; and
measuring a response of the GPCR polypeptide to the ligand.

11. A method for evaluating taste, comprising:
adding a test substance to the GPCR polypeptide expressed by the method according to any one of claims 1 to 7; and
measuring a response of the GPCR polypeptide to the test substance, wherein
the GPCR polypeptide is a taste receptor polypeptide.

12. A method for evaluating and/or selecting a suppressor of odor of a ligand for a GPCR of interest, comprising:
adding a test substance and a ligand for a GPCR of interest to the GPCR polypeptide expressed by the method according to any one of claims 1 to 7; and
measuring a response of the GPCR polypeptide to the ligand, wherein
the GPCR polypeptide is a trace amine-associated receptor polypeptide.

13. A method for evaluating and/or selecting a suppressor of odor of a ligand for a GPCR of interest, comprising:
adding a test substance to the GPCR polypeptide expressed by the method according to any one of claims 1 to 7; and
measuring a response of the GPCR polypeptide to the test substance, wherein
the GPCR polypeptide is a trace amine-associated receptor polypeptide.

14. The method according to any one of claims 8 to 13, wherein the response of the GPCR polypeptide is measured through intracellular cAMP level measurement by ELISA or reporter gene assay, calcium ion level measurement by calcium imaging or TGFα shedding assay, or potential change measurement inside and outside cell membranes by a two-electrode voltage clamp technique using *Xenopus* oocytes.

15. An altered GPCR polypeptide consisting of an amino acid sequence obtained by, in an amino acid sequence of a GPCR of interest (provided that an olfactory receptor is excluded), altering at least one amino acid residue different from that in a consensus amino acid sequence to an amino acid residue at a position corresponding thereto in the consensus amino acid sequence, wherein
the consensus amino acid sequence is an amino acid sequence derived by alignment of the amino acid sequence of the GPCR of interest and amino acid sequences of GPCRs encoded by orthologs of the GPCR of interest in vertebrates.

16. The altered GPCR polypeptide according to claim 15, wherein the consensus amino acid sequence is an amino acid sequence comprising consensus residues identified in accordance with the following criteria (i) to (iii) from the alignment:
(i) at each amino acid position of the alignment,
(i-i) when there exists one amino acid residue which is different from the amino acid residue of the GPCR of interest and has a frequency of appearance of 50% or more, the amino acid residue is identified as a consensus residue,
(i-ii) when there exist two amino acid residues having a frequency of appearance of 50%, the amino acid residue of the GPCR of interest is identified as a consensus residue,
(i-iii) when there exists an amino acid residue in the GPCR of interest and there exists no amino acid residue having a frequency of appearance of 40% or more, the absence of a consensus residue is identified,
(i-iv) when there exists no amino acid residue in the GPCR of interest and there exists an amino acid residue having a frequency of appearance of 60% or more, an amino acid residue having the highest frequency of appearance is identified as a consensus residue, and when there exist two or more amino acid residues having the highest frequency of appearance, an amino acid residue having the smallest molecular weight among the amino acid residues is identified as a consensus residue, and
(i-v) if none of the above (i-i) to (i-iv) is appropriate, the amino acid residue of the GPCR of interest is identified as a consensus residue;
(ii) when the consensus residues are identified in accordance with the criterion (i) and when a consensus residue nearest to the N terminus is a consensus residue at a position corresponding to the N terminus of the GPCR of interest or a C-terminal side thereof and is not a methionine residue, a consensus residue on an N-terminal side of a consensus residue consisting of a methionine residue positioned nearest to the N terminus is changed to the absence of a consensus residue; and
(iii) when the consensus residues are identified in accordance with the criterion (i) and when a consensus residue nearest to the N terminus is a consensus residue at a position corresponding to an N-terminal side of the N terminus of the GPCR of interest and is not a methionine residue, an amino acid residue having the highest frequency of appearance is identified as a consensus residue by tracing back one by one amino acid positions on an N-terminal side of the position of the consensus residue of the alignment until a methionine residue appears, and when there exist two or more amino acid residues having the highest frequency of appearance, an amino acid residue having the smallest molecular weight among the amino acid residues is identified as a consensus residue.

17. The altered GPCR polypeptide according to claim 15 or 16, wherein the GPCR of interest is a human GPCR.

18. The altered GPCR polypeptide according to any one of claims 15 to 17, wherein the GPCR polypeptide consists of an amino acid sequence obtained by, in an amino acid sequence of sequence identification number (2) of a GPCR (1) in the above Tables 1 and 2, altering at least one amino acid residue different from that in a consensus amino acid sequence of sequence identification number (3) to an amino acid residue at a position corresponding thereto in the consensus amino acid sequence.

19. The altered GPCR polypeptide according to claim 18, which consists of an amino acid sequence of any of SEQ ID NOs: 108 to 142, 144 to 214, and 275 to 312.

20. An altered GPCR polypeptide consisting of an amino acid sequence obtained by, in the amino acid sequence of SEQ ID NO: 36 of human TAAR6, altering at least one amino acid residue different from that in the consensus amino acid sequence of SEQ ID NO: 143 to an amino acid residue at a position corresponding thereto in the consensus amino acid sequence.

21. The altered GPCR polypeptide according to claim 20, which consists the amino acid sequence of SEQ ID NO: 143.

22. A polynucleotide encoding the altered GPCR polypeptide according to any one of claims 15 to 21.

23. A vector or a DNA fragment comprising the polynucleotide according to claim 22.

24. A transformed cell comprising the vector or the DNA fragment according to claim 23.
